(19) Europäisches Patentamt
European Patent Office
Office européen des brevets

(11) EP 3 263 706 A1

(12) EUROPEAN PATENT APPLICATION

(43) Date of publication:
03.01.2018 Bulletin 2018/01

(51) Int Cl.:
*C12N 15/113* (2010.01) *C07K 16/28* (2006.01)

(21) Application number: 16305785.4

(22) Date of filing: 28.06.2016

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicants:
- CENTRE NATIONAL DE LA RECHERCHE SCIENTIFIQUE -CNRS-
75016 Paris (FR)
- INSTITUT NATIONAL DE LA SANTE ET DE LA RECHERCHE MEDICALE (INSERM)
75013 Paris (FR)
- Universite Paris-Sud
91400 Orsay (FR)
- UNIVERSITE PARIS DESCARTES
75006 Paris (FR)

(72) Inventors:
- SAGNE, Corinne
75014 Paris (FR)
- VERDON, Quentin
75013 Paris (FR)
- GASNIER, Bruno
75005 Paris (FR)

(74) Representative: Regimbeau
20, rue de Chazelles
75847 Paris Cedex 17 (FR)

# (54) AGENTS TARGETING SNAT7 FOR TREATING CELLULAR METABOLISM REPROGRAMMING-ASSOCIATED DISEASES

(57) The present application relates to therapeutic agent reducing the expression or the activity of the SNAT7 transporter, encoded by the *SLC38A7* gene or one of its variants for use in the treatment of a cellular metabolism reprogramming-associated disease, preferentially a cancer depending on glutamine, more preferentially a cancer depending on glutamine activated by one or more oncogenes selected from Ras, Myc and Src, even more preferentially a cancer depending on glutamine activated by Ras. The present invention also relates to a pharmaceutical composition comprising at least one therapeutic agent for use according to the invention, or at least one expression vector according expressing said therapeutic agent for use to the invention.

EP 3 263 706 A1

SEQUENCE LISTING

<110> CENTRE NATIONAL DE LA RECHERCHE SCIENTIFIQUE (CNRS)
INSTITUT NATIONAL DE LA SANTE ET DE LA RECHERCHE MEDICALE (INSERM)
UNIVERSITE PARIS-SUD
UNIVERSITE PARIS DESCARTES

<120> AGENTS TARGETING SNAT7 FOR TREATING CELLULAR METABOLISM REPROGRAMMING-ASSOCIATED DISEASES

<130> B72959D36393

<160> 18

<170> PatentIn version 3.5

<210> 1
<211> 23
<212> DNA
<213> Artificial Sequence

<220>
<223> CRISPR-Cas9 guide RNAs

<400> 1
gtgcctctgt ccagaccccc agg 23

<210> 2
<211> 23
<212> DNA
<213> Artificial Sequence

<220>
<223> CRISPR-Cas9 guide RNAs

<400> 2
atcgtcgtca acgcgtgcct ggg 23

<210> 3
<211> 25
<212> DNA
<213> Artificial Sequence

<220>
<223> DsiRNA

<400> 3
agcacagaag uagcccaaac ucutc 25

<210> 4
<211> 27
<212> DNA
<213> Artificial Sequence

<220>
<223> DsiRNA

<400> 4
gaagaguuug ggcuacuucu gugcugg 27

<210> 5
<211> 25
<212> DNA
<213> Artificial Sequence

<220>
<223> DsiRNA

<400> 5
ccauagcuaa uagacauuuc ccagg 25

<210> 6
<211> 27
<212> DNA
<213> Artificial Sequence

<220>
<223> DsiRNA

<400> 6
ccugggaaau gucuauuagc uauggga 27

<210> 7
<211> 25
<212> DNA
<213> Artificial Sequence

<220>
<223> DsiRNA

<400> 7
caggucuaau guuuacaaac ggugc 25

<210> 8
<211> 27
<212> DNA
<213> Artificial Sequence

<220>
<223> DsiRNA

<400> 8
gcaccguuug uaaacauuag accuggu 27

<210> 9
<211> 84
<212> DNA
<213> Artificial Sequence

<220>
<223> SNAT7 inactivation - HeLa KO 2.22

<400> 9
gggaagcctc tcctgggggt ccggacagag gcaccacttc cacacttggg gccatcttca 60

tcgtcgtcaa cgcgtgcctg ggtg 84

<210> 10
<211> 289
<212> DNA
<213> Artificial Sequence

<220>
<223> SNAT7 inactivation - HeLa KO 1-24

<400> 10
gggaagcctc tcctgggggt ccggacagag gcaccacttc cacacttggg gccatcttca 60

tcgtcagaat gtcttcatat tatgaaagta tgtatcacat attcttatag agatgttttg 120

gtactcaaac tgtgtattat gtgcggtaca taacatatca aagtacccat actatatagt 180

atgtactgta cttaaaatat caaaataccc aaaatatata ttatatacgt tacataaact 240

atcaaataca caaagtatgt attatatact gtcaacgcgt gcctgggtg 289

<210> 11
<211> 78
<212> DNA
<213> Artificial Sequence

<220>
<223> SNAT7 inactivation - MIA PaCa-2 #34B allele1

<400> 11
gggaagcctc tcctgggcca tcacttccac acttggggcc atcttcatcg tcgtcaacgc 60

gtgcctgggt gcagggtt 78

<210> 12
<211> 93
<212> DNA
<213> Artificial Sequence

<220>
<223> SNAT7 inactivation - MIA PaCa-2 #34B allele2

<400> 12
gggaagcctc tcctgggctt ggggccatct tgaggcacct tccacacttg gggccatctt 60

catcgtcgtc aacgcgtgcc tgggtgcagg gtt 93

<210> 13
<211> 71
<212> DNA
<213> Artificial Sequence

<220>
<223> SNAT7 - MIA PaCa-2 #34I allele1

<400> 13
gggaagcctc tcctgggggt ctggacagag gcaccacttc cacacttggg gccgtgcctg 60

ggtgcagggt t 71

<210> 14
<211> 64
<212> DNA
<213> Artificial Sequence

<220>
<223> SNAT7 inactivation - MIA PaCa-2 #34I allele2

<400> 14
gggaagcctc tcctgggggt ctggacagag gcaccacttc cacacttggg gccatcttca 60

ggtt 64

<210> 15
<211> 78
<212> DNA
<213> Homo sapiens

<220>
<221> misc_feature
<223> SNAT7 genomic sequence (wild-type MIA PaCa2)

<400> 15
gggaagcctc tcctgggggt ctggacagag gcacggggcc atcttcatcg tcgtcaacgc 60

gtgcctgggt gcagggtt 78

<210> 16
<211> 29
<212> PRT
<213> Homo sapiens

<220>
<221> misc_feature
<223> SNAT7 protein sequence (wild-type MIA PaCa2)

<400> 16

Glu Ala Ser Pro Gly Gly Leu Asp Arg Gly Thr Thr Ser Thr Leu Gly
1 5 10 15

Ala Ile Phe Ile Val Val Asn Ala Cys Leu Gly Ala Gly
20 25

<210> 17
<211> 84
<212> DNA
<213> Homo sapiens

<220>
<221> misc_feature
<223> SNAT7 genomic sequence (wild-type HeLa)

<400> 17
gggaagcctc tcctgggggt ccggacagag gcaccacttc cacacttggg gccatcttca 60

```
tcgtcgtcaa cgcgtgcctg ggtg                                            84


<210>  18
<211>  27
<212>  PRT
<213>  Homo sapiens


<220>
<221>  misc_feature
<223>  SNAT7 protein sequence (wild-type HeLa)

<400>  18

Glu Ala Ser Pro Gly Gly Leu Asp Arg Gly Thr Thr Ser Thr Leu Gly
1               5                   10                  15


Ala Ile Phe Ile Val Val Asn Ala Cys Leu Gly
            20                  25
```

## Description

### TECHNICAL FIELD OF THE INVENTION

The present invention is in the field of diseases associated with cellular metabolism reprogramming. It relates to a therapeutic agent for use in the treatment of a cellular metabolism reprogramming-associated disease wherein said therapeutic agent reduces the expression or the activity of the SNAT7 transporter of sequence ID NO 1, encoded by the SLC38A7 gene or one of its variants. It further relates to a pharmaceutical composition comprising at least one said therapeutic agent for use in the treatment of disease associated with cellular metabolism reprogramming and a screening method for a therapeutic agent for use in the treatment of disease associated with cellular metabolism reprogramming.

### BACKGROUND OF THE INVENTION

The present invention is directed to therapeutic agents reducing the expression or the activity of the SNAT7 transporter, encoded by the *SLC38A7* gene or one of its variants for use in the treatment of diseases associated with cellular metabolism reprogramming, such as cancer depending on glutamine or immune diseases characterized by excessive M2 polarization of macrophages.

It is now known that tumor tissue is composed of cancer cells and surrounding stromal cells with diverse genetic/epigenetic backgrounds, a situation known as intra-tumoral heterogeneity. Cancer cells are surrounded by a totally different microenvironment than that of normal cells. Consequently, tumor cells must rapidly exhibit adaptive responses to hypoxia and hypo-nutrient conditions. This phenomenon of changes of tumor cellular bioenergetics, called "metabolic reprogramming", is required for both malignant transformation and tumor development, including cell invasion and metastasis.

Certain oncogenes, such as Myc, Ras and Src, can induce oncogenic transformations and increase Glutamine utilization in tumors. Because of oncogene-mediated metabolic reprogramming, cancer cells tend to exhibit "glutamine addiction" (Timmerman et al. 2013, Dang et al. 2009). This is a typical example of metabolic reprogramming in cancer cells with oncogene-addiction (Weinsteine et Joe 2008), suggesting a potential "Achilles' heel" of tumor cells that are addicted to glutamine metabolism in a manner that is mediated for example by Myc or Ras.

Indeed, glutamine is important to all cell types: it is the most abundant amino acid in blood and can be easily incorporated in many cellular metabolic pathways. Because of rapid anabolic processes, cancer cells have higher nutritional need than normal cells. To fulfil these needs, they mostly rely on glucose and free glutamine, but glutamine has a special role as it brings nitrogen as well as carbon and energy, contrary to glucose. Nitrogen is necessary for the anabolism of both proteins and nucleobases (Boroughs and DeBerardinis 2015) and glutamine is an important intermediate of these metabolic pathways.

Up to some extent, glutamine is necessary for cancer cell survival and depriving cancer cells from glutamine is a promising axis in the development of new therapies (White 2013). This however seems to depend on the type of cancer.

Like all proteinogenic amino acids, glutamine can be supplied to cell metabolism by several routes: (i) uptake from the extracellular environment by plasma membrane transporters; (ii) biosynthesis by glutamine synthase; recycling from endogenous proteins degraded by either (iii) the proteasome or (iv) the autophagy/lysosomal pathway; (v) and finally, a non-canonical supply route consisting in the internalization and lysosomal degradation of extracellular proteins, apoptotic corpses or live cells (entosis) (Pavlova and Thompson 2016). It should be noted that routes (iv) and (v) both depend on the export of proteolysis-derived glutamine from the lysosome to the cytosol by lysosomal transporters.

Plasma membrane uptake of free glutamine by the transporters ASCT2, SNAT1 or SNAT2 is often coupled to cancer growth (Bhutia et al 2015; Broer et al 2016). However, angiogenesis in tumours is often untidy, leading to parts of tumours to be badly irrigated by blood in oxygen, but as well in nutrients (Folkman and Shing 1992), so the overexpression of plasma membrane transporters can in some cases prove insufficient.

Glutamine synthesis by glutamine synthetase has been proved as an important actor of cancer cell metabolism (Cadoret et al. 2002).

Recently, cancer cells have also been shown to rely on the lysosomal degradation of proteins to meet their needs in glutamine. These can be intracellular proteins coming from autophagic processes (for survival under nutrient deficiency rather than cell growth ; (Strohecker et al. 2013)) or extracellular proteins obtained by macropinocytosis (Commisso et al. 2013). This last process is especially important in the nutrition of K-Ras activated cancer cells. This is of particular interest since these are the types of cancers that have a particularly bad prognosis today, such as pancreatic carcinoma.

In this context, the applicant surprisingly found that SNAT7 transporter is the primary route for lysosomal glutamine export in several cell types and that agents reducing the expression or the activity of the SNAT7 are able to starve cancer cells, preventing their growth in low glutamine environment, and/or tumors, thereby reducing and/or eliminating said cancer cells and/or said tumors.

Contrary to the present invention, previous study from Hagglung et al. (2011) described SNAT7 as a transporter for glutamine, histidine and alanine, with the best transport signal shown obtained with arginine. This preliminary study concludes that SNAT7 transporter may play a role at the plasma membrane of neurons in the glutamine-glutamate cycle between neurons and astrocytes, which may provide insight into key events in neurobiology, including neuronal metabolism and neurotransmitter cycling. However, the results provided in that study actually do not allow to clearly, directly and unambiguously characterize SNAT7 as a transporter for glutamine as they only showed a weak uptake of tritiated glutamine, with inconclusive experiments having very low signal-to noise ratio (as pointed out by Bröer 2014). In conclusion, this prior art teaching was definitely insufficient to undoubted fully assign to SNAT7 a role in glutamine transport.
SNAT7 transporter is encoded by the SLC38 gene family which comprises eleven members (SLC38A/SNAT 1-11).
Transporters of the SLC38 family are found in all cell types of the body. Some have been functionally characterized and mediate Na(+)-dependent net uptake and efflux of small neutral amino acids. As a result they are particularly expressed in cells that grow actively, or in cells that carry out significant amino acid metabolism, such as liver, kidney and brain. SLC38 transporters occur in membranes that face intercellular space or blood vessels, but do not occur in the apical membrane of absorptive epithelia. Members of the SLC38 family are highly regulated in response to amino acid depletion, hypertonicity and hormonal stimuli. SLC38 transporters play an important role in amino acid signalling and have been proposed to act as transceptors (that is a transporter-like protein that transduces signals in response to substrate binding) independent of their transport function (Bröer 2014).
Among them, 7 members have been functionally characterized as secondary active amino acid transporters: SNAT1-5 and SNAT8-9 (Bröer 2014; Wang et al. 2015; Rebsamen et al. 2015; Hagglund et al. 2015). SNAT1, 2, 4 and 8 are amino acid transporters with broad substrate selectivity. They are expressed at the plasma membrane. They have been referred to as system A transporters, a term based on early biochemical studies of amino acid transport (Christensen 1990), which means they are able to transport small neutral amino acids, like alanine or proline. In addition, they can as well transport glutamine, asparagine, cysteine or histidine (Bröer 2014). SNAT 3 and 5 have narrower substrate selectivity, transporting mostly glutamine, arginine and histidine. The two proteins have distinct tissue expression profile: for instance, neurons and glial cells express mostly SNAT3 and kidney cells SNAT5 (Bröer 2014). SNAT9 was recently characterized as a key component of amino acid sensing at the lysosomal membrane (J. Jung, Genau, and Behrends 2015; S. Wang et al. 2015; Rebsamen et al. 2015). Moreover, studies provided evidence for its ability to act as a signalling protein, and for a lysosomal transporter function, having as main substrates glutamine and arginine.

## DESCRIPTION

The present invention is directed to a therapeutic agent for use in the treatment of a cellular metabolism reprogramming-associated disease wherein said therapeutic agent reduces the expression or the activity of the SNAT7 transporter of sequence ID NO 1, encoded by the *SLC38A7* gene or one of its variants.
By "cellular metabolism reprogramming-associated disease" it is refered herein to diseases related to cells or a group of cells that modify their metabolism to adapt to a new microenvironment compared to normal cells, or to respond to environmental cues.
The term "therapeutic agent" means an agent that induce a desired, beneficial, often pharmacological, effect upon administration to a human or an animal, whether alone or in combination with other pharmaceutical excipients or inert ingredients.
Preferentially, cellular metabolism reprogramming-associated disease is a cancer-associated cellular metabolism reprogramming, preferentially a cancer depending on glutamine, more preferentially a cancer depending on glutamine activated by one or more oncogenes selected from Ras, Myc and Src, even more preferentially a cancer depending on glutamine activated by Ras.
The term "treating" or "treatment" means stabilizing, alleviating, curing, or reducing the progression of the cancer.
The term "variant" means that two nucleotide or amino acid sequences, when optimally aligned share at least 70% sequence identity, or at least 80% sequence identity, or at least 85% sequence identity, or at least 90% sequence identity, or 95% sequence identity or more (e.g., 99% sequence identity or more). For sequence comparison, typically one sequence acts as a reference sequence (e.g., parent sequence), to which test sequences are compared.
One example of algorithm that is suitable for determining percent sequence identity and sequence similarity is the BLAST algorithm, which is described in Altschul et al., J. Mol. Biol. 215:403 (1990). Software for performing BLAST analyses is publicly available through the National Center for Biotechnology Information (publicly accessible through the National Institutes of Health NCBI interne server). Typically, default program parameters can be used to perform the sequence comparison, although customized parameters can also be used. For amino acid sequences, the BLASTP program uses as defaults a wordlength (W) of 3, an expectation (E) of 10, and the BLOSUM62 scoring matrix (Henikoff et al., 1989).
The term "cancer depending on glutamine activated by Ras" means cancer wherein the oncogene Ras is activated, and wherein cancer cells have been shown to rely on the degradation of proteins to meet their needs in glutamine, especially on extracellular proteins obtained by macropinocytosis (Commisso et al. 2013).

Preferentially, the cancer depending on glutamine activated by Ras is selected from pancreatic cancer, colon cancer, biliary tract cancer, small intestine cancer, lung cancer, cervical cancer, glioma, glioblastoma, ovarian cancer, melanomas, leukemias, lymphomas, soft tissue sarcomas, testis seminomas and thyroids carcinomas, preferentially pancreatic cancer, colon cancer, lung cancer, glioma, glioblastoma and ovarian cancer more preferentially pancreatic cancer, colon cancer, lung cancer, glioma, glioblastoma and ovarian cancer.

In another embodiment, the cellular metabolism reprogramming-associated disease is selected from autoimmunity, innate immunity or adaptive immunity diseases associated with a cellular metabolism reprogramming. It has been shown that a specific phenotypic adaptation of activated macrophages, known as 'alternative macrophage polarization' or 'M2 polarization', involves metabolic reprogramming and enhanced glutamine biosynthesis, and that M2 macrophage function requires high glutamine supply (Jha AK et al, 2015). Owing to the high phagocytic activity of macrophages and the increased expression of SNAT7 in M2 macrophages relative to M1 macrophages, SNAT7 should significantly contribute to the cytosolic availability of glutamine and impairing SNAT7 should alter the M1/M2 balance of macrophage polarization towards the M1 state.

Therapeutic agents inhibiting the expression or activity of SNAT7 may thus be used for the treatment of immune disease associated with imbalance of the M1 vs. M2 macrophage polarization towards M2, such as asthma; skin allergy; autoimmune diseases, (for instance, systemic lupus erythematosus); chronic stress associated with modern life; M2 promotion of infectious diseases; and the contribution of M2 macrophages to tumor growth and vascularization.

In a preferred embodiment, the therapeutic agent according to the invention is selected from inhibitor of SNAT7 transporters, such as anti-SNAT7 antibody, anti-SNAT7 aptamer, CRISPR/Cas9 guide RNAs or CRISPR-Cas9 single guide RNA directed to SNAT7 gene, ribozymes and any kind of RNA interference (RNAi) directed to SNAT7 mRNA, such as Dicer-substrate short interfering RNAs (DsiRNAs), Small interfering RNA (siRNA), Antisense RNA (asRNA), RNA interference (RNAi), small hairpin RNA (shRNA) and micro RNA (miRNA).

As used herein, "CRISPR-Cas9" refers to a two component ribonucleoprotein complex with guide RNA and a Cas9 endonuclease. CRISPR refers to the Clustered Regularly Interspaced Short Palindromic Repeats type II system used by bacteria and archaea for adaptive defense. This system enables bacteria and archaea to detect and silence foreign nucleic acids, e.g., from viruses or plasmids, in a sequence-specific manner (Jinek, M., et al. (2012) Science 337(6096):816-21). In type II systems, guide RNA interacts with Cas9 and directs the nuclease activity of Cas9 to target DNA sequences complementary to those present in the guide RNA. Guide RNA base pairs with complementary sequence in target DNA. Cas9 nuclease activity then generates a double- stranded break in the target DNA.

In bacteria, Cas9 polypeptides bind to two different guide RNAs acting in concert: a CRISPR RNA (crRNA) and a trans-activating crRNA (tracrRNA). The crRNA and tracrRNA ribonucleotides base pair and form a structure required for the Cas9-mediated cleavage of target DNA. However, it has been demonstrated that a single guide RNA (sgRNA) may be engineered to form the crRNA:tracrRNA structure and direct Cas9-mediated cleavage of target DNA (Jinek, M., et al. (2012) Science 337(6096):816-21). Moreover, since the specificity of Cas9 nuclease activity is determined by the guide RNA, the CRISPR-Cas9 system has been explored as a tool to direct double-stranded DNA breaks in heterologous cells, enabling customizable genome editing (Mali, P., et al. (2013) Science 339(6121):823-6).

Accordingly, in some embodiments, sequences of CRISPR-Cas9 guide RNAs are SEQ ID NO 1: GTGCCTCTGTCCAGACCCCCAGG and SEQ ID NO 2: ATCGTCGTCAACGCGTGCCTGGG.

As used herein, "CRISPR-Cas9 single guide RNA" (the terms "single guide RNA" and "sgRNA" may be used interchangeably herein) refers to a single RNA species capable of directing Cas9-mediated cleavage of target DNA. In some embodiments, a single guide RNA may contain the sequences necessary for Cas9 nuclease activity and a target sequence complementary to a target DNA of interest.

In the context of the invention, **"RNA interference"** (hereinafter referred to as RNAi) is interpreted as a process by which a double stranded RNA (dsRNA) with a given sense nucleic sequence leads to the breakdown of all messenger RNA (mRNA) comprising said nucleic sequence, in a manner specific to said nucleic sequence and inhibit the targeted genes.

The process of RNAi can be achieved using small interfering RNA (or siRNA). These siRNAs are dsRNA of fewer than 30 nucleotides comprising in their sense sequence a sequence that is highly homologous, preferably identical, to a fragment of the targeted mRNA. When a siRNA crosses the plasma membrane, the reaction of the cell is to destroy the siRNA and all the sequences comprising an identical or highly homologous sequence. Thus a mRNA with a fragment that is identical or highly homologous to the siRNA sequence will be destroyed, the expression of this gene being thus inhibited.

shRNA may be also used as inhibitor according to the present invention. As used herein, an "shRNA molecule" includes a conventional stem-loop shRNA, which forms a precursor miRNA (pre-miRNA). "shRNA" also includes micro-RNA embedded shRNAs (miRNA-based shRNAs), wherein the guide strand and the passenger strand of the miRNA duplex are incorporated into an existing (or natural) miRNA or into a modified or synthetic (designed) miRNA. When transcribed, a conventional shRNA forms a primary miRNA (pri-miRNA) or a structure very similar to a natural pri-miRNA. The pri-miRNA is subsequently processed by Drosha and its cofactors into pre-miRNA. Therefore, the term "shRNA" includes pri-miRNA (shRNA-mir) molecules and pre-miRNA molecules.

Dicer-substrate short interfering RNAs (DsiRNAs) may also be used as inhibitor according to the present invention. DsiRNAs are chemically synthesized 27mer duplex RNAs that have increased potency in RNA interference compared to traditional, 21 mer siRNAs. In a preferred embodiment, DsiRNA are selected from:

- SEQ ID 3: rArGrCrArCrArGrArArGrUrArGrCrCrCrArArArCrUrCrUTC and SEQ ID 4: rGrArArGrArGrUrUrUrGrGrGrCrUrArCrUrUrCrUrGrUrGrCrUrGrG;
- SEQ ID 5: rCrCrArUrArGrCrUrArArUrArGrArCrArUrUrUrCrCrCrAGG and SEQ ID 6: rCrCrUrGrGrGrArArArUrGrUrCrUrArUrUrArGrCrUrArUrGrGrGrA
- SEQ ID 7: rCrArGrGrUrCrUrArArUrGrUrUrUrArCrArArArCrGrGrUGC and SEQ ID 8: rGrCrArCrCrGrUrUrUrGrUrArArArCrArUrUrArGrArCrCrUrGrGrU

As herein used the term **"antisense RNA"** relates to unmodified or chemically modified single-stranded DNA molecules which are relatively short in general and which is able to hybridize to a unique sequence in the total pool of targets present in cells, the sequence of said DNA molecule being complementary by virtue of Watson-Crick bp hybridization, to a specific mRNA and is able to inhibit said mRNA expression and then induce a blockade in the transfer of genetic information from DNA to protein.

To inhibit SNAT7 transporter expression and/or SNAT7 transporter activity, ribozymes may be also used. In this context, **"ribozymes"** also called **"catalytic RNAs"** or **"RNAzymes",** are RNA molecules that are capable of catalyzing specific biochemical reactions, similar to the action of protein enzymes. Ribozymes allow the cleavage or ligation of RNA and DNA and peptide bond formation. Examples of ribozymes include the hammerhead ribozyme, the VS ribozyme, Leadzyme and the hairpin ribozyme.

Aptamers may be also used in the present invention for inhibiting SNAT7 transporter expression and SNAT7 transporter activity. By **"aptamer"** is meant a ligand-specific DNA or RNA molecule with high affinity for a protein. Consequently, this meaning comprises "natural" aptamers and chemically-modified analogs.

Aptamers are selected by the alternation of selection and amplification, which makes it possible to direct the evolution of the population in a Darwinian manner: in the population, the most "apt" molecules are selected, hence the origin of the name "aptamers" given to oligonucleotides having the desired feature, stemming from the selection. Standard genetic engineering techniques (cloning, sequencing, expression) make it easy to identify these aptamers individually, then to characterize them and produce them in large amounts.

Aptamers can be selected by means of an optimized *in vitro* selection protocol known as systemic evolution of ligands by exponential enrichment (SELEX) (WO 91/19813).

The SELEX method makes it possible to generate in large amounts ligands of very high affinity and specificity. This approach rests on exposing the target molecule to a library of potential ligands. A system of desorption/selection cycles makes it possible to enrich the population of ligands that most specifically interact with the target molecule. The final population obtained is then isolated and characterized, allowing its large-scale resynthesis.

Aptamers can be oligodeoxynucleotides (DNA) or oligoribonucleotides (RNA). RNA and DNA aptamers having comparable characteristics were selected against the same target. Additionally, both compounds are competitive inhibitors of one another, suggesting overlapping interaction sites.

Preferably, the aptamer according to the invention can bind with high affinity to SNAT7 transporter, and most preferably to the specific inhibiting site of SNAT7 transporter.

By "high affinity" is meant, in the context of the present invention, a specific interaction of the aptamer with the target and a dissociation constant that is sufficiently low to allow significant inhibition of the catalytic activity of the enzyme.

Antisense oligonucleotides, interfering RNAs, ribozymes and aptamers may be made of natural or modified DNA or RNA.

In a preferred embodiment of the invention, anti-SNAT7 transporter antibodies or antigen-binding fragments thereof may be used for inhibiting SNAT7 transporter activity.

Particularly, said anti- SNAT7 transporter antibody or antigen-binding fragment thereof may be selected from polyclonal antibodies, monoclonal antibodies, bispecific antibodies, and from fragments selected from Fv, scFv, Fab, F(ab')2, Fab', scFv-Fc, diabodies, and any antigen-binding fragment whose half-life has been increased by chemical modification.

The term "antibody" is used herein in the broadest sense and specifically covers monoclonal antibodies (including full length monoclonal antibodies) of any isotype such as IgG, IgM, IgA, IgD, and IgE, polyclonal antibodies, multispecific antibodies, chimeric antibodies, and antigen-binding fragments. An antibody reactive with a specific antigen can be generated by recombinant methods such as selection of libraries of recombinant antibodies in phage or similar vectors, or by immunizing an animal with the antigen or an antigen-encoding nucleic acid.

In one embodiment, the present application relates to polyclonal antibodies as inhibitors of SNAT7 transporter activity.

A **"polyclonal antibody"** is an antibody which was produced among or in the presence of one or more other, non-identical antibodies. In general, polyclonal antibodies are produced from a B-lymphocyte in the presence of several other B-lymphocytes producing non-identical antibodies. Usually, polyclonal antibodies are obtained directly from an immunized animal.

According to preferred embodiment, the anti-SNAT7 transporter antibody, or antigen-binding fragments thereof, used in the invention is a monoclonal antibody.

As used herein, the term **"monoclonal antibody"** refers to an antibody arising from a nearly homogeneous antibody population. More particularly, the individual antibodies of a population are identical except for a few possible naturally-occurring mutations which can be found in minimal proportions. In other words, a monoclonal antibody consists of a homogeneous antibody population arising from the growth of a single cell clone (for example a hybridoma, a eukaryotic host cell transfected with a DNA molecule coding for the homogeneous antibody, a prokaryotic host cell transfected with a DNA molecule coding for the homogeneous antibody, etc.) and is generally characterized by heavy chains of one and only one class and subclass, and light chains of only one type. Monoclonal antibodies are highly specific and are directed against a single antigen.

Moreover, the antibodies used in the present invention may be chimeric or humanized antibodies, or antigen-binding fragments, which can be obtained by genetic engineering or by chemical synthesis.

The term "chimeric antibody" as used herein refers to an antibody containing a natural variable region (light chain and heavy chain) derived from an antibody of a given species in combination with constant regions of the light chain and the heavy chain of an antibody of a species heterologous to said given species. Thus, a "chimeric antibody", as used herein, is an antibody in which the constant region, or a portion thereof, is altered, replaced, or exchanged, so that the variable region is linked to a constant region of a different species, or belonging to another antibody class or subclass. "Chimeric antibody" also refers to an antibody in which the variable region, or a portion thereof, is altered, replaced, or exchanged, so that the constant region is linked to a variable region of a different species, or belonging to another antibody class or subclass.

As used herein, the term **"humanized antibody"** refers to a chimeric antibody which contain minimal sequence derived from non-human immunoglobulin. In one embodiment, a humanized antibody is a human immunoglobulin (recipient antibody) in which residues from a CDR of the recipient are replaced by residues from a CDR of a non-human species (donor antibody) such as mouse, rat, rabbit or non-human primate having the desired specificity, affinity, and/or capacity. In some instances, framework ("FR") residues of the human immunoglobulin are replaced by corresponding non-human residues. Furthermore, humanized antibodies may comprise residues that are not found in the recipient antibody or in the donor antibody. These modifications may be made to further refine antibody performance, such as binding affinity. In general, a humanized antibody will comprise substantially all of at least one, and typically two, variable domains, in which all or substantially all of the hypervariable loops correspond to those of a non-human immunoglobulin sequence, and all or substantially all of the FR regions are those of a human immunoglobulin sequence, although the FR regions may include one or more individual FR residue substitutions that improve antibody performance, such as binding affinity, isomerization, immunogenicity, etc. The number of these amino acid substitutions in the FR is typically no more than 6 in the H chain, and in the L chain, no more than 3. The humanized antibody optionally will also comprise at least a portion of an immunoglobulin constant region (Fc), typically that of a human immunoglobulin.

According to a preferred embodiment of the invention, the anti-SNAT7 transporter antibody is a bispecific monoclonal antibody.

For inhibiting SNAT7 transporter expression and/or SNAT7 transporter activity, an antigen-binding antibody fragment may also be used. An **"antigen-binding antibody fragment"** comprises a portion of an intact antibody that maintains binding to the antigen, preferably the antigen binding and/or the variable region of the intact antibody. Examples of antibody fragments include Fab, Fab', $F(ab')_2$and Fv fragments; diabodies; linear antibodies (see U.S. Pat. No. 5,641,870, Example 2; Zapata et al., 1995); single-chain antibody molecules and multispecific antibodies formed from antibody fragments. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain ($V_H$), and the first constant domain of one heavy chain ($C_H1$). Each Fab fragment is monovalent with respect to antigen binding, i.e., it has a single antigen-binding site. Pepsin treatment of an antibody yields a single large **$F(ab')_2$** fragment which roughly corresponds to two disulfide linked Fab fragments having different antigen-binding activity and is still capable of cross-linking antigen. Fab' fragments differ from Fab fragments by having a few additional residues at the carboxy terminus of the $C_H1$ domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known

Further inhibitors of SNAT7 transporter expression and/or SNAT7 transporter activity, in addition to those described above, may be obtained by implementation of classical screening methods well known to skilled artisans.

In a preferred embodiment, the therapeutic agent for use according to the invention is a siRNA molecule, the sequence thereof being selected from SEQ ID NO: 3 to SEQ ID NO: 8, or CRISPR/Cas9 molecules of SEQ ID NO: 1 and/or 2.

Another object of the present invention relates to an expression vector comprising a nucleic acid sequence expressing said therapeutic agent for use according to the invention.

The term "expression vector" may refer to any molecular structure conveying genetic information. The genetic information may be encoded as deoxyribonucleic acid (DNA) (double stranded DNA (dsDNA), single stranded DNA (ssDNA)), ribonucleic acid (RNA), (single-stranded RNA (ssRNA), double-stranded RNA (dsRNA)), or as a DNA analog. The vector according to the invention may be any vector known in the art such as, e.g., a linear vector, a circular vector, a viral vector or a bacterial vector.

In another aspect, the invention relates to a host cell comprising said vector according to the invention.

By "host cell", it is referred herein to a prokaryotic or eukaryotic cell, capable of replicating the nucleic acid coding for the mutated fluorescent protein according to the invention or the recombinant vector as previously described, and thus capable of expressing the mutated fluorescent protein of the invention. A host cell can be "transfected" or "transformed" according to a process known to the skilled person in the art by means of which said nucleic acid or said vector is transferred or introduced into the host cell. Examples of such methods include, without limitation, electroporation, lipofection, and calcium phosphate transfection.

The inhibitors of SNAT7 transporter expression or SNAT7 transporter activity as described above may be also included in pharmaceutical composition for treating a cellular metabolism reprogramming-associated disease according to the invention.

In another aspect, the invention thus relates to pharmaceutical composition comprising at least one said therapeutic agent for use according to the invention, or at least one said expression vector according to the invention.

In some embodiments, the pharmaceutical composition according to the invention further comprises at least one additional anticancer agent in combination, either sequentially or simultaneously.

According to the invention "anticancer agent" refers to agent commonly used in art for treating tumors or tumor metastases in a patient.

In a particular embodiment, the pharmaceutical composition according to the invention further comprises at least one pharmaceutically acceptable carrier, diluent, excipient and/or adjuvant, for use in the treatment of a cellular metabolism reprogramming-associated disease.

This pharmaceutically acceptable carrier can be a solid or liquid and the type is generally chosen based on the type of administration being used. The anticancer agent can be coadministered in the form of a tablet or capsule, as an agglomerated powder or in a liquid form. Examples of suitable solid carriers include lactose, sucrose, gelatin and agar. Capsule or tablets can be easily formulated and can be made easy to swallow or chew; other solid forms include granules, and bulk powders. Tablets may contain suitable binders, lubricants, diluents, disintegrating agents, coloring agents, flavoring agents, flow-inducing agents, and melting agents. Examples of suitable liquid dosage forms include solutions or suspensions in water, pharmaceutically acceptable fats and oils, alcohols or other organic solvents, including esters, emulsions, syrups or elixirs, suspensions, solutions and/or suspensions reconstituted from non-effervescent granules and effervescent preparations reconstituted from effervescent granules. Such liquid dosage forms may contain, for example, suitable solvents, preservatives, emulsifying agents, suspending agents, diluents, sweeteners, thickeners, and melting agents. Parenteral and intravenous forms would also include minerals and other materials to make them compatible with the type of injection or delivery system chosen.

Preferably, the pharmaceutical composition of the invention may contain, in addition to the inhibitor of SNAT7 transporter expression or SNAT7 transporter activity as described above, various diluents, fillers, salts, buffers, stabilizers, solubilizers, and other materials well known in the art.

In still another aspect, the present invention relates to a method for treating a cellular metabolism reprogramming-associated disease, comprising administering an inhibitor of SNAT7 transporter expression or a pharmaceutical composition according to the invention as described above.

In another aspect, the invention relates to a kit comprising :

a) at least one therapeutic agent for use according to the invention; and/or
b) at least one expression vector according to the invention; and/or
c) at least one host cell according to the invention; and/or
d) a pharmaceutical composition according to the invention.

In another aspect, the invention relates to a screening method for a therapeutic agent for use in the treatment of disease associated with cellular metabolism reprogramming comprising the steps of:

a) providing SNAT7 protein;
b) contacting said SNAT7 protein with a therapeutic agent candidate;
c) determining the effect of said therapeutic agent candidate on the SNAT7 protein glutamine transport activity;
d) selecting said therapeutic agent candidate if it significantly reduces or inhibits SNAT7 protein glutamine transport activity as determined in step c).

The term "screening method" as used herein means a method using a polypeptide or a cell expressing a polypeptide used as a subject to be brought into contact with a test substance, for identifying an agent reducing or inhibiting SNAT7 protein glutamine transport activity.

In a preferred embodiment in said screening method for a therapeutic agent according to the invention, said disease associated with cellular metabolism reprogramming is a cancer-associated with cellular metabolism reprogramming, preferentially is a cancer depending on glutamine, more preferentially cancer depending on glutamine activated by the oncogenes Ras, Myc or Src, more preferentially by the oncogene Ras or Myc, even more preferentially by the oncogene Ras.

In another aspect, the invention relates to a method of treatment of cancer comprising the steps of:

a) Determining from a biological sample of a patient suffering from a cancer or susceptible to suffer from cancer, if said cancer is associated to cellular metabolism reprogramming, preferentially cancer depending on glutamine, more preferentially cancer depending on glutamine activated by the oncogenes Ras, Myc or Src, more preferentially by the oncogene Ras or Myc, even more preferentially by the oncogene Ras;
b) If yes, then administering said therapeutic agent for use according to the invention to said patient.

The "biological sample" relates to a tumor sample, preferably a tumor tissue biopsy or whole or part of a tumor surgical resection. The sample may be collected according to conventional techniques and used directly for diagnosis or stored.

**DESCRIPTION OF THE FIGURES**

**Figure 1. SNAT7 is necessary to sustain BSA-dependent growth in low free glutamine environment.** MIA-PaCa2 (MP2) cells can obtain glutamine from Bovine Serum Albumin (BSA) to sustain cell proliferation (A). MIA-PaCa2 cells grew for 5 days in medium with controlled concentrations of glutamine, with (triangle) or without (circle) BSA. At lower concentrations, when the cellular division rate was slowed by the lack of glutamine, BSA accelerated cell division. In contrast, when glutamine was sufficient in the medium, it had no effect. This experiment was performed to replicate published results (for instance, Commisso et al, Nature 2013), and was used as a basis to analyze the nutritive role of SNAT7.
Silencing of SNAT7 expression reduces BSA-dependent growth in low free glutamine environment (B). MIA-PaCa2 cells were transfected with dsiRNAs targeted to human SNAT7 or HPRT1 ('control 2') or without any target in the human and murine genomes ('control 1') and tested for SNAT7 expression and for growth as in A. Upper picture: Cell lysates were analysed by Western Blot using an antibody against SNAT7. dsiRNAs snat7-1, -2 and -3 drastically reduced SNAT7 levels when compared to controls. Lower graph: MIA-PaCa2 were treated with three different dsiRNAs targeting hSNAT7 (dark grey bars) or with control dsiRNAs (white bars). Two days after the start of silencing, they were transfected with a plasmid encoding either mouse SNAT7 (stripes), which is insensitive to hSNAT7 dsiRNAs, or with a negative control (sialin P334R, no stripes). Cells were seeded in control medium with 0.2 mM glutamine with or without BSA. The BSA-dependent growth was calculated after 3 days. Silencing of SNAT7 significantly reduces BSA-dependent growth. Conversely, mSNAT7 expression restores growth in silenced cells.
Genetic inactivation of SNAT7 abolishes BSA-dependent growth in low free glutamine environment (C). Alignment: SNAT7 expression was fully suppressed in the MIA PaCa-2 cell line using the CRISPR/Cas9 approach. The panel shows genomic sequences for both SNAT7 gene alleles in two 'knock-out '(KO) cell clones obtained with this approach and, for comparison (sequence), the wild-type genomic and protein sequences. The SNAT7 mutations obtained (frameshifts and amino acid deletion) are predicted to induce complete loss-of-function. **Figure D:** lysates from WT cells or cells KO for SNAT7 were analyzed by Western Blot using an antibody against SNAT7. No SNAT7 can be detected in KO cells. **Figure E:** The same protocol as Fig 1A (circle, without BSA; triangle, with BSA) was repeated on three MIA-PaCa2 clones, one control (CTB) and two KO clones (34B and 341) transfected with the mSNAT7 or a control lysosomal transporter (sialin) before growth analysis. At low concentrations of glutamine, BSA rescued the proliferation of control cells, but it had absolutely no effect on KO cells except if they re-express SNAT7. **Figure F:** Quantification of BSA effect on cell growth at 0.2 mM free extracellular glutamine. The two graphs show that SNAT7 is necessary and sufficient for BSA-dependent growth of MIA-PaCa2 cells in low free glutamine environment.

**Figure 2. A novel, live-cell assay of lysosomal amino acid transport suggests that SNAT7 selectively exports glutamine and asparagine from lysosomes.**

Principle of the novel live-cell assay developed in our laboratory (A). The assay is based on the use of TFEB, a transcription factor which regulates lysosomal biogenesis, and on the use of chemical tools to artificially overload lysosomes with amino acids. TFEB upregulates expression of most lysosomal genes, but it is also part of homeostatic mechanism that senses excessive accumulation of macromolecules or small molecules in lysosomes and activates lysosomal biogenesis to adapt the pool of lysosomes to the degradative needs of the cell. Under normal conditions, TFEB is inactive and localized in the cytosol in most cells (left scheme). When cells are challenged with an amino acid ester (AAE), the compound easily enters the cell and its organelles. Fast ester hydrolysis within lysosomes induces strong overload of the corresponding amino acid. This overload is in turn detected by the cell. Consequently, TFEB is activated and translocated to the nucleus (middle scheme). If this experiment is repeated on cells which overexpress a cognate lysosomal amino acid exporter, lysosomal overload and, consequently, TFEB nuclear translocation are reduced (right scheme). Conversely, if the lysosomal transporter imports the tested amino acid into the lysosome, TFEB nuclear translocation should be increased (not depicted). Screening of SNAT7 for amino acid transport using the novel live-cell assay (**B**). Picture: Representative micrograph of HeLa cells expressing mRFP-hTFEB and challenged with an amino acid ester. TFEB localization was classified in three different groups according the predominant mRFP-hTFEB distribution: nuclear ("N"), cytosolic ("C") or mixed ("M"). **Figure C:** HeLa cells overexpressing mRFP-hTFEB and either EGFP-SNAT7 ('S') or PQLC2-EGFP ('P'), a lysosomal exporter for cationic amino acids, were incubated with diverse AAEs at optimal concentrations determined in preliminary experiments for 2 hrs. They were then fixed, imaged and scored for TFEB localization (> 50 cells for at least 10 fields per condition). Results are displayed as a stacked bar graph where black, dark-grey and light-grey bars represent predominantly nuclear, mixed and cytosolic TFEB distribution, respectively. Cells exposed to no compound (ctrl) or to high sucrose (sucr) provided negative and positive controls, respectively. Cells overexpressing PQLC2 selectively resisted to artificial lysosomal lysine overload, in agreement with the substrate selectivity of this transporter and the live-cell assay rationale. In contrast, SNAT7-overexpressing cells resisted to artificial lysosomal overload of asparagine and glutamine, but not of other tested amino acids, strongly showing that SNAT7 selectively exports asparagine and glutamine from lysosomes.

**Figure 3. SNAT7 is the primary lysosomal glutamine transporter.**

Genetic inactivation of SNAT7 in the HeLa cell line using the CRISPR/Cas9 approach (A). The alignment shows the shows genomic sequences of two selected homozygous KO clones and, for comparison, the wild-type sequence. The frameshift and deletion/insertion found in the KO alleles are predicted to induce complete loss-of-function. Picture: in agreement with this prediction, Western blot analysis failed to detected significant SNAT7 immunoreactivity in the KO clones.

Native SNAT7 localizes to lysosomes (**B**). The intracellular distribution of SNAT7 in wild-type HeLa cells were analyzed by subcellular fractionation using two types of centrifugation: differential and isopycnic in a continuous sucrose gradient (left scheme). The distribution of SNAT7, as determined by western blotting, was compared to that of compartment-specific enzymes (remaining graphs). In both types of separation, SNAT7 co-migrated with the lysosomal marker.

Principle of the counter-transport assay (**C**). To facilitate SNAT7 activity measurement, an exchange reaction was assayed on crude lysosomal fractions between internal and external substrates instead of the net export of amino acids occurring under physiological conditions. Lysosomes (left scheme) were artificially loaded with unlabeled glutamine using glutamine tert-butyl ester (middle scheme). They were then washed and incubated with radiolabelled glutamine (right scheme). In most transporters, the presence of a high substrate concentration on one side of the membrane stimulates ('trans-stimulates') uptake of substrate from the other side. This behavior ('counter-transport' towards the high substrate compartment) reflects the existence of partial transport reaction cycles and is independent of the direction of the full transport cycle under natural conditions. According to this general property of transporters, SNAT7 trans-stimulation by high luminal glutamine should result in an uptake of radioactive glutamine into the lysosome.

SNAT7 is the main lysosomal transporter for glutamine (**D**). Lysosomal fractions were loaded with glutamine using an ester precursor as in **C**. They were then washed and incubated with [3H]-glutamine. Radiolabelled glutamine accumulated linearly over time in fractions from WT HeLa cells (black squares). This uptake occured into lysosomes, and not into contaminating organelles of the crude lysosomal fractions, since selective osmotic lysis of lysosomes by a high concentration of glycine methyl-ester completely released the radioactivity. The uptake of radiolabelled glutamine is barely detected in subcellular fractions from SNAT7 KO cells (grey squares).

Same experiment as in figure 3D performed on fractions prepared from three types of HeLa cells: control; SNAT7 KO; or SNAT KO cells transfected with a plasmid expressing mSNAT7 (E). mSNAT7 expression rescued lysosomal glutamine uptake.

**Figure 4. SNAT7 is a lysosomal transporter of glutamine and asparagine.**

Lysosomal distribution of native SNAT7 in HeLa cells (**A**). HeLa cells from a control (CT2) and a SNAT7 KO clone (34.1.24) were fractionated according to protocol of fig 3B. Fractions were analysed by Western Blot using an antibody against SNAT7. Most of SNAT7 is recovered in fraction L in the control clone and there is no SNAT7 immunoreactivity in the SNAT7 KO clone.

Glutamine and asparagine share a common lysosomal transporter (**B**). The same protocol as fig 3D was applied, but fractions were loaded with esters of glutamine or asparagine, and tested for uptake of tritiated asparagine or glutamine. Both internal glutamine and internal asparagine stimulated countertransport, and both amino acids were accumulated in a lysosome-specific manner, showing that the underlying transporter has dual amino acid specificity.

SNAT7 KO does not alter lysosomal sensitivity to overload-induced osmotic lysis (C). Lysosomal fractions from WT and KO cells were incubated with increasing concentrations of glycine-methyl-ester and tested for lysosomal integrity using a latency enzymatic assay. The activity of the lysosomal enzyme beta-galactosidase was measured in the absence or in the presence of Triton X100. The percentage of intact lysosomes was determined as the fraction of detergent-sensitive activity. Wild-type and KO lysosomes showed identical dose-response curves, thus excluding enhanced vulnerability as a possible cause of defective countertransport in KO lysosomes.

**Figure 5. Lysosomal pH dependence of SNAT7 activity.**

SNAT7 activity depends on ATP (A). The same protocol as figure 3D was replicated in the absence or presence of 5 mM ATP: Mg2+.

SNAT7 activity requires an acidic lysosomal lumen (**B**). The same protocol as fig 3D was applied in the presence of ionophores to selectivity alter the pH and/or electrical components of the lysosomal H+ gradient. Left panel: Predicted effects of each tested ionophore on the pH gradient and membrane potential across the lysosomal membrane. Right panel: Effect of the ionophores on SNAT7 activity. Disruption of the pH gradient (nigericin or FCCP), but not of the membrane potential (valinomycin), impairs glutamine countertransport.

**Figure 6. SNAT7 substrate selectivity is restricted to glutamine and asparagine on both sides.**

The experiment of **figure 3D** was repeated, but with 10 radiolabelled amino acids applied to glutamine-loaded lysosomes across 2 independent experiments.

**Figure 6A** shows combined data from the two experiments. Specific transport was calculated as follows:

$$\%\,uptake_{amino\ acid} = 100\ \times \frac{uptake\ 30min_{amino\ acid} - uptake\ lysis_{amino\ acid}}{uptake\ 30min_{Gln} - uptake\ lysis_{gln}}$$

**Figure 6B** shows the results of a representative experiment. Arginine did not accumulate into the subcellular fractions. Asparagine and glutamine accumulated in a lysosome-dependent manner. Valine accumulated to high level, yet in a lysosome independent manner since glycine methylester-induced lysis (L) had not effect. Only glutamine and asparagine were transported by SNAT7 from the cytosolic side.

Substrate selectivity of SNAT7 on its luminal side **(Figure 6C).** The experiment of figure 3D was repeated with different amino acid esters, thus loading lysosomes with the corresponding amino acids. Only internal glutamine and asparagine could activate glutamine countertransport by SNAT7, showing it is selective for these two amino acids on its luminal side.

## EXAMPLES

### *Materials and Methods*

Cell culture and transfection

All cells but neurons were kept at 37°C in 5%$C0_2$ and in glucose rich DMEM supplemented with 100 units/mL penicillin, 100 μg/mL streptomycin and 10% fetal bovine serum.

For electroporation or HeLa cells, a GHT 1287 electroporator (Jouan) was used. 2000000 cells were resuspended in 50 μL ice-cold PBS supplemented with 5-10 μg DNA. Immediately after mixing, cells were subjected to ten square pulses (200 V, 3ms) at 1 Hz with 5mm-spaced electrodes. Cells were then resuspended and distributed in fourteen wells (24-wells plate).

Cells samples in 24 wells plates were rinsed with ice-cold PBS++ (PBS supplemented with 0.1 mM $MgCl_2$ and 0.1 mM $CaCl_2$). They were resuspended in Laemmli buffer at 1,250,000 cells per mL. The equivalent of 250,000 cells was analyzed by SDS-Page (10% gel)

Separated proteins were elecrotransfered onto nitrocellulose. The membrane was blocked for 1 h in PBS supplemented with 5% non-fat-milk, then incubated in the primary antibody (see "antibodies") for 1h in PBS supplemented with Tween20 0.05% and milk 5%. After three rinse of 5 minutes in PBS+Tween, the membrane was incubated with horseradish-conjugated secondary antibody in PBS+Tween+milk for 1h, and then rinsed three times. Immune complexes were detected using Lumi Light Plus (Roche) and images acquired using an Image Quant LAS 4000 (GE Healthcare

TFEB-based lysosome overload live cell assay

HeLa cells were electroporated with DNA encoding mRFP-TFEB and EGFP-fused transporters and seeded onto coverslips 48 h prior to experiment. Cells were treated for 2h with complete medium supplemented with 100 mM sucrose or esters: alanine methyl-ester, 3 mM; glutamate dimethyl-ester 10 mM, glycine methyl-ester 4.5 mM, lysine methyl-ester 40 mM, leucine methyl-ester 1.7 mM, methionine methyl-ester 6 mM, asparagine methyl-ester 27 mM, glutamine tert-butyl-ester 3.75 mM, serine methyl-ester 3.7 mM, tyrosine methyl-ester 7 mM. Cells were then fixed with paraformaldehyde 4% in PBS and coverslips were mounted with Fluoromount-G.

For each condition, at least 50 cells were observed using a 40X objective with an Eclipse TE-2000 (Nikkon) and divided in three categories depending on TFEB localization: C (cytosolic, N (nuclear), M (mixed). Images were analyzed using Image J.

SNAT7 edition with the CRISPR-Cas9 approach

We used the protocol from Ran, Nature Prot, 2013. pSpCas9n(BB)-2A-Puro was used. This plasmid encodes a mutated form of Cas9 that catalyzed simple-strand breaks. To trigger deletion-insertions in the target gene, two sites are selected that are close enough from one another. This helps reduce the number of off-targets. Sites were selected using www.crispr.mi.edu. The region targeted is localized in exon 1 of *SNAT7:* (129-194). Clones were validated by PCR on genomic DNA of cells and by Western Blot with the anti-SLC38A7 antibody from Atlas Antibodies.

DQ-red-BSA uptake

MIA PaCa-2 cells were incubated for 30 minutes in DMEM supplemented with 50μg/mL DQ-red-BSA (Life Technologies). They were then washed three times with PBS++ (PBS supplemented with 0.1 mM $CaCl_2$ and $MgCl_2$) and incubated for two hours in complete, fresh medium before being processed for flow cytometry.

Flow cytometry

Cells were prepared in the dark. They were dissociated using trypsin. There were then centrifuged at 300 g. Then, they were washed with PBS supplemented with EDTA 5mM, and centrifuged again. The pellet was resuspended in 150 μL PBS+EDTA. Then, 150μL 4% PFA in PBS was added. Cells were gently mixed for 10 minutes in PFA before being analyzed or kept at 4°C overnight.

Cells were analyzed at the Service commun de cytométrie en flux (CUSP, Université Paris Descartes, France) using a Canto II Becton Dickinson. If necessary, cells were diluted with PBS+EDTA.

Cellular fractionation

HeLa cells were fractionated by differential centrifugation essentially as described by de Duve et al., Biochem. J, 1955. This allows a partial separation of the different cellular organelles into six fractions: E, Extract or post-nuclear supernatant; N, Nuclear fraction; M, Heavy mitochondrial fraction; L, Light mitochondrial fraction; P, microsomal fraction and S, Soluble fraction. Note that in our adapted protocol, all ultracentrifugation steps were conducted at 4°C using a 70.1Ti rotor and that the M fraction (3.5 mL per tube) was pelleted at 10 000 rpm for 3'15", then washed (2 mL final volume), and re-centrifuged for 2'24" at 10 000 rpm. A M + L pooled fraction was then centrifuged in a linear sucrose density gradient as described previously (Wattiaux, Biochem J, 2010). Enzyme activities of lysosomal ß-galactosidase, mitochondrial cytochrome oxidase, peroxisomal catalase, plasma membrane alkaline phosphodiesterase, and of the endoplasmic reticulum marker alkaline α-glucosidase were measured as described (Gasingirwa, Biochem J, 2010; Puissant, Traffic, 2014; Beaufay, J. Cell Biol., 1974; Delta Valle, Mol Cell Proteome, 2011).

Transport assay on lysosomal preparations

22,500,000 HeLa cells were dissociated with trypsin and centrifuged 10min at 300g. They were washed twice in 1.125 mL purification buffer (250 mM sucrose, 10 mM triethanolamine, 10 mM $CH_3COOH$, 1mM EDTA, 2.5mM KCl, 5mM MgATP, Pierce protease inhibitor cocktail 1X, adjusted at pH7.60). Unless mentioned, all the rest of this protocol is done at 4°C. For lysis, cells were passed 50 times is a 0.25 mM needle using a syringe.

Lysates (fraction L) were centrifuged at 750 g for 10 min. Supernatants were kept and pellets resuspended in 1.125 mL purification buffer. Pellets were centrifuged again in the same way and supernatants from both centrifuges pooled (fraction S1). Pellets (C1) were discarded. S1 was centrifuged at 1500 g for 15 minutes. Pellets (C2) were discarded and supernatants (S2) centrifuged at 20000 g for 15 minutes. Supernatants (S3) were discarded and Pellets (C3) resuspended in 125μL purification buffer.

For transport, C3 fractions were supplemented with amino acid esters (same concentrations as in TFEB test) and incubated for 15 minutes at 25°C. They were split into 5 tubes and centrifuged at 20000 g for 15 minutes. Supernatants were discarded. Each tube was resuspended in 400 μL purification buffer supplemented with 1.27 μCi of radioactive amino acids (Perkin-Elmer, 50 Ci/mmol). Each tube was then split into seven hemolysis tubes (50 μL per tube). One tube was immediately filtered. Three tubes were filtered after 30 minutes at 25°C. The last three tubes were supplemented with 100mM glycine methyl-ester at 30 minutes and filtered at 40 minutes.

Lysosomal suspensions were filtered on vaccum using 0.45 μm nitrocellulose filters (Millipore). First, 3 mL ice-cold PBS were added to lysosomal suspensions, and then filtered. Tubes were then rinsed twice with 3mL ice-cold PBS and the rinsing liquid was filtered onto the same filter. Radioactivity on filters was counted in Emulsifier-Safe cocktail (Packard) with a Tri-Carb 2100 TR liquid scintillation analyser (Packard).

Latency test

C3 lysosomal fractions were diluted 5 times in purification buffer (see transport assay on lysosomal fractions). Reaction was mixed at 4°C as follows: sample: 20 μL; methylumbelliferyl-galactoside-N-acetyl-galactoside 40 mM 2.5 μL; triton X-100: 0 or 5 μL; NaCl 0.4M 25 μL; $NaCH_3COO$ 0.2M pH 5.0 50 μL, water 102.5 or 97.5 μL respectively. Samples were incubated at 37°C for 10 minutes. The reaction was then stopped by adding 1.7 mL ice-cold $Na_2CO_3$ 0.5 M + glycine 0.5 M pH 10.5. Fluorescence was analyzed using a Fluorescence Spectrophotometer F-7100 (Hitachi) (excitation at 350nm, emission 450nm).

Growth assays

For growth assays, MIA PaCa-2 or A2780 cells were seeded at respectively 15,000 and 25,000 cells per well in 24-wells plates in glutamine-free DMEM (Gibco), supplemented with dialyzed fetal serum (Gibco, 10%), penicillin (100 units/mL) and streptomycin (100μg/mL), glutamine (Sigma) and delipidified BSA (Calbiochem, 2%). Medium was changed daily.
Cells were dissociated after growth using trypsin and resuspended in complete medium. They were finally counted using a Coulter Z2.

***Results***

SNAT7 is necessary to sustain BSA-dependent growth in low free glutamine environment

To study the nutrition of cancer cells, MIA-PaCa2 cells were used by the inventors. This cell line derives from a human pancreatic carcinoma. It is activated by K-Ras and it was used by Commisso and colleagues (Commisso et al. 2013), who proved this cell line to be especially dependent on macropinocytosis to meet its need in free glutamine-restricted conditions.
First, the dependency of MIA-PaCa2 to glutamine was assessed at low concentrations. Cells were seeded at a fixed concentration of glutamine. They grew for five days and were counted, out of which their average number of mitosis was calculated. Half of the cells were as well supplemented with delipidified BSA to check whether extracellular proteins could help cells cope better with the lack of glutamine by providing glutamine through the macropinocytosis pathway.
Results in Figure 1A show that MIA-PaCa2 division rate is heavily affected by the concentration of glutamine in the medium. However, division rates were increased at low glutamine concentration when BSA was added to the medium. At high concentrations of glutamine, it had however no effect, showing that the growth stimulation effect of extracellular BSA is mediated by the supply of glutamine.
Since BSA could be used as a source of glutamine and since it had been shown that lysosomes were important in this process, SNAT7 was expected to be important to export glutamine from lysosomes after the degradation of BSA. Thus, SNAT7 expression was reduced by RNA interference and the impact of BSA on MIA-PaCa2 proliferation rate measured. The importance of SNAT7 to sustain BSA-dependent proliferation was then measured at 0.2 mM glutamine, the concentration with the highest BSA effect.
Results in **Figure 1B** show that human SNAT7 expression was reduced in MIA-PaCa2 cells using dsiRNAs targeting three different regions of SNAT7 mRNAs. In addition, a rescue was performed with the murine form of SNAT7. This form was insensitive to silencing. The reduction of expression due to silencing was checked at the level of the protein. The inventors generated cell lysates and analysed them by Western Blot using our 144 antibody against SNAT7.The same growth protocol was then applied to these cells and the BSA-dependent proliferation was calculated.

Results in **Figure 1B** show that DsiRNAs were quite efficient to reduce the expression of SNAT7, as shown in Western Blot. Concerning the proliferation assays, in negative controls, BSA increased cell proliferation by 50%. However, when SNAT7 was silenced, BSA had significantly lower effect left on cell proliferation (25, 20 and 17% for each dsiRNA respectively), showing that SNAT7 sustains BSA-dependent growth, presumably by supplying lysosome-derived glutamine to the cell.

Similar results were obtained with another cell line: A2780. These cells are derived from a human breast carcinoma. Interestingly, they are not activated by KRas, showing that the supply of glutamine through macropinocytosis and lysosomal degradation of extracellular proteins when free glutamine is insufficient could be a more generic response to nutritional stress than previously anticipated.

DsiRNAs only induce a reduction in the expression of target genes. To confirm the former results and get clearer responses, the same protocol was applied to MIA-PaCa2 cells KO for SNAT7 generated using the CRISPR/Cas-9 technique: the loss of expression was analysed by Western Blot and the effect of BSA on the growth of these cells under a limiting concentration of glutamine was analyzed **(Figure 1C).**

The Western Blot **(Figure 1D)** confirmed that no residual expression of SNAT7 could be observed in KO models. In these cells, strikingly, the genetic inactivation of SNAT7 abolished the BSA dependent growth, showing that SNAT7 is the main, if not only, transporter supplying glutamine for growth from the macropinocytosis/lysosomal pathway **(Figure 1 F).**

<u>A novel, live-cell assay of lysosomal amino acid transport suggests that SNAT7 selectively exports glutamine and asparagine from lysosomes.</u>

This assay enables to measure indirectly transport activity at the lysosomal level, and not at the plasma membrane, in intact cells. It was performed on HeLa cells exposed to amino acid esters. Because of their being more hydrophobic than regular amino acids, these esters are able to diffuse through membranes. However, lysosomes are the major organelles containing hydrolases, which are able to catalyse the cleavage of the ester bond and thus generate the free amino acid. Once inside lysosomes, non-modified amino acids are trapped and their efflux through transporters is slow, hence creating a strong overload inside lysosomes. It should be pointed out that other organelles, such as ER, contain hydrolases as well, but most of the accumulation occurs inside lysosomes.

We speculated that this overload could be reduced when a transporter able to export some of the overloaded amino acid was overexpressed. Eventually, control cells expressing a mock construct should display high levels of the accumulated amino acid, whereas cells expressing a transporter able to export the overloaded amino acid should have a smaller accumulation. **(Figure 2A)** The level of lysosomal amino acid overload should thus reflect transport activity of the tested protein.

To detect lysosomal overload, we used the transcription factor TFEB, which controls most of the genes implicated in autophagy and lysosome biogenesis. Under normal conditions, TFEB usually is localized in cytosol, but it is targeted to the nucleus in case of lysosomal overload (Sardiello et al. 2009). Thus, in cells with no overexpressed transporter, overload should be quite strong and TFEB quite dramatically translocated to the nucleus, whereas this translocation should be milder in cells overexpressing the corresponding transporter.

Results in **Figure 2B** show the TFEB localisation by epifluorescence, after a plasmid encoding mRFP-TFEB was generated and transfected intro HeLa cells. Indeed, despite a few publications, antibodies against endogenous TFEB are not highly specific and precise localisation is difficult to assess.

It is important to point out that HeLa cells used for the following experiments were used 48h after transfection, as transfection results in lysosomal stress (especially with lipofectants which accumulate into lysosomes) and, consequently, in TFEB translocation. 24h after transfection, TFEB was indeed almost always fully translocated to nuclei in HeLa cells. Besides, electroporation was favoured over lipofection, for lysosomal stress was noticed to be much milder then. In the end, 48h after electroporation, mRFP-TFEB was mostly localized in cytosol of HeLa cells **(Figure 2B).**

When overexpressed in HeLa cells fused to EGFP, SNAT7 was sorted to lysosomes. Thus the results in **Figure 2C** show the characterization of amino acid selectivity of SNAT7 with our TFEB-AAE assay. PQLC2-EGFP was used as a control, like previously. As expected, TFEB is translocated to nucleus in about 50% of PQLC2-expressing cells in all conditions but when they were treated with lysine-methyl-ester, confirming its function of lysosomal lysine transporter. Similarly, EGFP-SNAT7 had no effect on the nuclear distribution of TFEB except when cells were treated with glutamine and asparagine esters, which suggested SNAT7 is a lysosomal transporter selective for glutamine and asparagine.

SNAT7 is a the primary lysosomal glutamine transporter.

To determine more direct evidence for the localization of native SNAT7, the inventors characterized an antibody against human SNAT7, recognizing the first 53 amino acids. The specificity of this antibody was assessed by Western Blot on cellular lysates of WT HeLa cells or SNAT7 KO HeLa cells, which were generated using the CRISPR/Cas-9 method (Ran et al. 2013). Results in **Figure 3A** show that the antibody recognized a 40 kDa band in WT cells, but no in SNAT7 KO cells.

Other bands recognized by this antibody were present in both types of cell **(Figure 3A).** This band had an apparent mass lower than the predicted mass of human SNAT7 (50kDa), but this phenomenon often happens for highly polytopic proteins due to an excessive binding of SDS compared to less hydrophobic proteins. Thus, the antibody used is specific for SNAT7 and that only the band at 40 kDa should be considered.

To determine the subcellular localization of native SNAT7, WT HeLa cells were fractionated into five fractions by differential centrifugations presented in **Figure 3B.** The inventors measured the activity of beta-galactosidase, a lysosomal marker, in each fraction. Beta galactosidase was mostly found in fraction L, with a little amount in fractions M and P. The inventors measured as well the activity of other enzymes representative for other compartments: cytochrome oxidase for mitochondria, alkaline phosphodiesterase for plasma membrane, alkaline a-diesterase for ER and catalase for peroxysomes. Most enzymes were not enriched in the same fractions as beta-galactosidase, except perhaps for phosphodiesterase, the marker for plasma membrane, although it was much less enriched in fraction L **(Figure 3B).** Then, these fractions were analysed by Western Blot using the SNAT7 antibody. We obtained a very similar profile to the one of betagalactosidase: most of SNAT7 was found in fraction L, a little in fractions P and M. The strong enrichment in fraction L correlated much better with the distribution of betagalactosidase (lysosomal enzyme) than with the one of phosphodiesterase (plasma membrane enzyme). The fact that beta-galactosidase and SNAT7 had a similar behavior along this fractionation strengthened the hypothesis of a lysosomal localization of endogenous SNAT7 **(Figure 3B).** For a further confirmation, fractions M and L were pooled and loaded onto a sucrose gradient, which helped separating organites further. After isopycnic centrifugation, the inventors obtained eleven fractions of different densities. They quantified betagalactosidase activity in each fraction, in addition to other enzymes representative for other compartments. Beta galactosidase was mostly found in fractions 7 and 8, around a density of 1.15, whereas almost all other markers, such as cytochrome oxydase, the enzyme marker for mitochondria, were found in denser fractions, around 1.2. Only the plasma membrane marker, alkaline phosphodiesterase, was found in the same fractions as beta-galactosidase consistently with other studies using isopycnic centrifugations to purify lysosomes, in which plasma membrane was a typical contaminant. Then, isopycnic centrifugation fractions were analyzed by Western Blot. Most of SNAT7 could be found in fractions 7 and 8, similarly to betagalactosidase and phosphodiesterase. **(Figure 3B).**

Drawing in **Figure 3C** show how inventors artificially loaded isolated lysosomes with specific amino acids using an ester precursor and then exposed them to radiolabelled amino acids to and measure the counter-transport activity of SNAT7.

More precisely, the following steps were followed to perform this counter-transport assay **(Figure 3C):**

- Load lysosomes, or not, with unlabelled glutamine,
- Expose the loaded and unloaded lysosomes to radiolabelled glutamine to measure their capacity to accumulate this radiotracer.

For the first step, lysosomal fractions were loaded with glutamine by taking advantage once again of amino acid esters, like in the TFEB-AAE assay. This made very advanced purifications of lysosomes unnecessary, since the treatment with esters selectively loads lysosomes but not, or poorly, contaminating organelles. The dose of glutamine tert-butylester used for loading (3.75mM) was chosen to avoid damaging lysosomes using a latency test.

For the second step, after treatment with the ester, lysosomal fractions were centrifuged, washed and incubated with radiolabelled glutamine. Based on the expected trans-stimulation property of SNAT7, prior loading with unlabelled glutamine should stimulate accumulation of radiolabelled glutamine into the lysosomes if this amino acid is translocated by SNAT7. After uptake, fractions were filtered and the accumulated radioactivity quantified.

This assay was applied to WT HeLa cells and to a SNAT7 KO HeLa cell clone obtained using the CRISPR/Cas-9 method. Results in **Figure 3D** show the accumulation of radioactivity over time for 30 minutes. After 30 minutes, lysosomes were specifically lysed using 100mM of glycine methyl-ester. This compound can as glutamine tert-butyl-ester enter lysosomes and be cleaved to generate glycine. However, at this concentration, its accumulation is so fast that it creates a hypo-osmotic shock. Other compartments are not or little affected, which allows discriminating between lysosomal uptake and uptake into contaminating organelles. Lysosomal fractions from WT cells took up far more radiolabelled glutamine than fractions from KO cells, in which very little uptake was detectable, proving that SNAT7 is indeed a lysosomal transporter for glutamine. Moreover, the marginal accumulation observed into KO lysosomes shows that SNAT7 represents the predominant glutamine pathway across the lysosomal membrane. When lysosomes were lysed, almost this entire uptake was lost, showing that this uptake took place in lysosomes and not in another compartment **(Figure 3D).**

To confirm and strengthen this result presented in **Figure 3E,** SNAT7 KO cells were transiently transfected by electroporation with a plasmid encoding EGFP-mSNAT7. This expression partially restored the ability of lysosomal fractions to take up radiolabelled glutamine by counter-transport, confirming that SNAT7 is a transporter for glutamine.

SNAT7 is a lysosomal transporter of glutamine and asparagine

**Figure 4A.** Control and KO HeLa cells have been lysed, submitted to differential centrifugations as depicted in Figure 3B and analyzed by western blotting with the antibody directed against SNAT7. In control cells, SNAT7 immunoreactivity is mostly present in the L fraction and no signal is found in the KO cells

**Figure 4B.** The same experiments as in Figure 3D show that lysosomal glutamine or asparagine preloading is necessary to drive the counter-transport of radiolabelled glutamine or asparagine inside lysosomal fractions.

Results in **Figure 4C** show that absence of glutamine counter-transport in SNAT7 KO cells is not due to this lysosomes being more sensitive to amino acid-ester loading osmotic stress. Lysosomal fractions from WT or KO cells were treated with increasing concentrations of glycine-methyl-ester in uptake buffer. Like all amino acid esters, it can go through the lysosomal membrane. There, lysosomal esterases generate the corresponding amino acid, which is trapped inside. If concentrations of ester are too high, this creates an osmotic shock, resulting in the breakdown of the lysosomal membrane. If lysosomes from KO cells are more fragile than lysosomes from WT cells, the dose-response curve should be shifted to the left. The integrity of lysosomes was measured by a latency assay using betagalactosidase. The principle of this assay is the following: beta-galactosidase activity is measured in fractions treated or not with Triton X-100, a detergent. In presence of the detergent, all beta-galactosidase can access to substrate.

Without it, beta-galactosidase in intact lysosomes cannot react with the substrate. Subtracting both activities thus gives access to how much of beta-galactosidase is inside intact lysosomes. For both fractions from WT and KO cells, the latency of betagalactosidase decreased with higher concentrations of glycine methyl-ester. They did so in a similar manner, indicating that SNAT7-defective lysosomes are not more sensitive to osmotic shock.

Lysosomal pH dependence of SNAT7 activity

The activity of SNAT7 was further studied and results were presented in **Figure 5A.** As an active transporter, SNAT7 would require energy, directly or indirectly, which is usually brought by ATP. Normal uptake buffer contained 5 mM ATP. So, the former experiment was replicated while removing ATP from the uptake buffer. This resulted in a strong decrease in the counter-transport of glutamine into lysosomes, showing that SNAT7 strongly depends on the activity of the V-type H+-ATPase of the lysosomal membrane. Results in **Figure 5B** show that SNAT7 activity depends on the chemical component of the lysosomal proton gradient. By translocating protons into the lysosome, the V-ATPase generates both a concentration (pH) gradient and a potential gradient, since protons carry a positive charge. To examine which components of the proton electrochemical gradient ‗drive' SNAT7, we applied three ionophores (5 μM) to the uptake buffer:

- FCCP, which abolishes both components of the gradient, since it is a proton ionophore.
- Nigericin, which abolishes the pH gradient, but not the potential gradient by exchanging protons for K+ in an electroneutral manner.
- Valinomycin, which abolishes the potential gradient only by letting K+ exit lysosomes.

FCCP completely abolished the uptake of glutamine inside lysosomal fractions, confirming that SNAT7 activity depends on the v-ATPase and the proton electrochemical gradient. Interestingly, only nigericin, and not valinomycin, replicated this effect. This showed that SNAT7 depended on the pH gradient and not on the potential gradient for its activity **(Figure 5B).** However, we cannot exclude that under physiological conditions (i.e.net glutamine export) SNAT7 could be modulated by the lysosomal potential, in contrast with the presumably electroneutral glutamine/glutamine exchange monitored in our artificial counter-transport assay.

SNAT7 substrate selectivity on its cytosolic side is restricted to glutamine and asparagine.

Concerning the selectivity on the cytosolic side, the same experiment was done; but lysosomes were loaded with glutamine while lysosomal fractions were incubated in various tritiated amino acids, which uptake was quantified. Nine amino acids were tested: alanine, arginine, asparagine, aspartate, glutamate, histidine, phenylalanine, proline and valine. These amino acids were selected to be representative samples of diverse chemical classes of amino acids.

Results in **Figure 6A and 6B** show that, in addition to glutamine, only asparagine could be taken up into lysosomes. All 8 other amino acids showed no specific uptake. Yet, for alanine and valine, a high uptake was observed into lysosomal fractions. However, this uptake was insensitive to glycine-methyl-ester, indicating that contaminating organelles were responsible for this uptake.

Results in **Figure 6C** show the SNAT7 selectivity for amino acids on its luminal side. First, to study the selectivity of SNAT7 on the luminal side, lysosomal fractions were treated with different amino acid esters to load them with different amino acids in their lumen. In addition to glutamine-tert-butyl-ester, alanine-methyl-ester, asparagine-methyl-ester, serine-methyl-ester and glutamate-dimethyl-ester were used. The side chain of these amino acids have distinct chemical natures to sample what family of amino acids could be transported by SNAT7. On the cytosolic side, radiolabelled glutamine was added like in former experiments.

Finally, the same test was used with asparagine-methyl-ester, to load lysosomes with asparagine, but fractions were then incubated with radiolabelled asparagine. An uptake of radiolabelled asparagine could be observed. This showed that SNAT7 can counter-transport both asparagine and glutamine in either direction.

Altogether, these results indicate that SNAT7 has the same selectivity on its cytosolic side and luminal sides: it is highly selective for asparagine and glutamine, and do not transport other amino acids, in particular arginine, in striking contrast with the broader substrate selectivity reported by Hagglung et al (Hagglund et al. 2011).

In addition to glutamine, only asparagine, but not alanine, serine or glutamate, could trigger SNAT7 countertransport activity on the luminal side. This showed that SNAT7 is quite specific for asparagine and glutamine on its luminal side.

**REFERENCES**

Dang CV, Le A, Gao P. MYC-induced cancer cell energy metabolism and therapeutic opportunities. Clin Cancer Res. 2009;15(21):6479-83.

Boroughs, L. K., & DeBerardinis, R. J. (2015). Metabolic pathways promoting cancer cell survival and growth. Nature Cell Biology, 17(4), 351-359.

Bhutia YD, Babu E, Ramachandran S, Ganapathy V. Amino Acid transporters in cancer and their relevance to "glutamine addiction": novel targets for the design of a new class of anticancer drugs. Cancer Res. 2015;75(9): 1782-8.

Bröer A, Rahimi F, Bröer S. Deletion of Amino Acid Transporter ASCT2 (SLC1A5) Reveals an Essential Role for Transporters SNAT1 (SLC38A1) and SNAT2 (SLC38A2) to Sustain Glutaminolysis in Cancer Cells. J Biol Chem. 2016;291(25):13194-205.

Cadoret A, Ovejero C, Terris B, Souil E, Levy L, Lamers WH, Kitajewski J, Kahn A, Perret C. New targets of beta-catenin signaling in the liver are involved in the glutamine metabolism. Oncogene. 2002 Nov 28;21(54):8293-301

Commisso C, Davidson SM, Soydaner-Azeloglu RG, Parker SJ, Kamphorst JJ, Hackett S, Grabocka E, Nofal M, Drebin JA, Thompson CB, Rabinowitz JD, Metallo CM, Vander Heiden MG, Bar-Sagi D. Macropinocytosis of protein is an amino acid supply route in Rastransformed cells. Nature. 2013;497(7451):633-7.

Folkman J, Shing Y. Angiogenesis J Biol Chem. 1992 Jun 5;267(16):10931-4

Hagglund MG, Sreedharan S, Nilsson VC, Shaik JH, Almkvist IM, Bäcklin S, Wrange 0, Fredriksson R. Identification of SLC38A7 (SNAT7) protein as a glutamine transporter expressed in neurons. J Biol Chem. 2011;286(23):20500-11.

Hagglund MG, Hellsten SV, Bagchi S, Philippot G, Löfqvist E, Nilsson VC, Almkvist I, Karlsson E, Sreedharan S, Tafreshiha A, Fredriksson R. Transport of L-glutamine, L-alanine, L-arginine and L-histidine by the neuron-specific Slc38a8 (SNAT8) in CNS. J Mol Biol. 2015;427(6 Pt B):1495-512.

Pavlova NN, ThompsonCB. The Emerging Hallmarks of Cancer Metabolism. Cell Metab. 2016 Jan 12;23(1):27-47.

Strohecker, A. M., Guo, J. Y., Karsli-Uzunbas, G., Price, S. M., Chen, G. J., Mathew, R.,White, E. (2013). Autophagy sustains mitochondrial glutamine metabolism and growth of BrafV600E-driven lung tumors. Cancer Discovery, 3(11), 1272-85. http://doi.org/10.1158/2159-8290.CD-13-0397

Timmerman LA, Holton T, Yuneva M, Louie RJ, Padró M, Daemen A, Hu M, Chan DA, Ethier SP, van 't Veer LJ, Polyak K, McCormick F, Gray JW. Glutamine sensitivity analysis identifies the xCT antiporter as a common triple-negative breast tumor therapeutic target.

Cancer Cell. 2013 Oct 14;24(4):450-65 Weinstein IB, Joe A. Oncogene addiction Cancer Res. 2008 May 1;68(9):3077-80 White, E. (2013). Exploiting the bad eating habits of Ras-driven cancers. *Genes & Development,* 27(19), 2065-71. http://doi.org/10.1101/gad.228122.113

Bröer, S. (2014). The SLC38 family of sodium-amino acid co-transporters. Pflugers Archiv European Journal of Physiology, 466, 155-172. http://doi.org/10.1007/s00424-013-1393-y Wang, S., Tsun, Z., Wolfson, R., Shen, K., Wyant, G., Plovanich, M., ... Sabatini, D. (2015).

Lysosomal amino acid transporter SLC38A9 signals arginine sufficiency to mTORC1, 347(6218), 188-194.

Rebsamen, M., Pochini, L., Stasyk, T., de Araújo, M. E. G., Galluccio, M., Kandasamy, R. K., ... Superti-Furga, G.

(2015). SLC38A9 is a component of the lysosomal amino acid sensing machinery that controls mTORC1. Nature, 519(7544), 477-81. http://doi.org/10.1038/nature14107
Christensen HN. Role of amino acid transport and countertransport in nutrition and metabolism. Physiol Rev. 1990 Jan;70(1 ):43-77
Jung, J., Genau, H. M., & Behrends, C. (2015). Amino Acid-Dependent mTORC1 Regulation by the Lysosomal Membrane Protein SLC38A9. Molecular and Cellular Biology, 35(14), 2479-2494. Ran, F. A., Hsu, P. D., Wright, J., Agarwala, V., Scott, D. a, & Zhang, F. (2013). Genome engineering using the CRISPR-Cas9 system. Nature Protocols, 8(11), 2281-308. http: //doi.org/10.1038/nprot.2013.143
Jha, A. K., Huang, S. C. C., Sergushichev, A., Lampropoulou, V., Ivanova, Y., Loginicheva, E., ... Artyomov, M. N. (2015). Network integration of parallel metabolic and transcriptional data reveals metabolic modules that regulate macrophage polarization. Immunity, 42(3), 419-430. http://doi.org/10.1016/j.immuni.2015.02.005

**Claims**

1. A therapeutic agent for use in the treatment of a cellular metabolism reprogramming-associated disease wherein said therapeutic agent reduces the expression or the activity of the SNAT7 transporter of sequence ID NO 1, encoded by the *SLC38A7* gene or one of its variants.

2. The therapeutic agent for use according to claim 1, wherein said a cellular metabolism reprogramming-associated disease is a cancer-associated cellular metabolism reprogramming, preferentially a cancer depending on glutamine, more preferentially a cancer depending on glutamine activated by one or more oncogenes selected from Ras, Myc and Src, even more preferentially a cancer depending on glutamine activated by Ras.

3. The therapeutic agent for use according to claim 1 or 2, wherein said cancer depending on glutamine activated by Ras is selected from pancreatic cancer, colon cancer, biliary tract cancer, small intestine cancer, lung cancer, cervical cancer, glioma, glioblastoma and ovarian cancer, preferentially pancreatic cancer, colon cancer, lung cancer, glioma, glioblastoma and ovarian cancer more preferentially pancreatic cancer, colon cancer, lung cancer, glioma, glioblastoma and ovarian cancer.

4. The therapeutic agent for use according to claim 1, wherein said a cellular metabolism reprogramming-associated disease is an autoimmunity or inflammatory disease associated with a cellular metabolism reprogramming.

5. The therapeutic agent for use according to any of the preceding claims, wherein said therapeutic agent is selected from inhibitor of SNAT7 transporter, such as anti-SNAT7 antibody, anti-SNAT7 aptamer, CRISPR/Cas9 guide RNAs or CRISPR-Cas9 single guide RNA directed to SNAT7 gene, and any kind of RNA interference (RNAi) directed to SNAT7 mRNA, such as Small interfering RNA (siRNA), Antisense RNA (asRNA), RNA interference (RNAi), small hairpin RNA (shRNA) and micro RNA (miRNA).

6. The therapeutic agent for use according to any of the preceding claims, wherein said therapeutic agent is a siRNA molecule, the sequence thereof being selected from SEQ ID NO 2, SEQ ID NO 3 and/or SEQ ID NO 4, or CRISPR/Cas9 molecule of SEQ ID NO X5, or an antibody anti-SNAT7 selected from polyclonal antibodies, monoclonal antibodies or bispecific antibodies.

7. An expression vector comprising a nucleic acid sequence expressing said therapeutic agent for use according to claim 5 or 6.

8. A host cell comprising said vector according to claim 7.

**9.** A pharmaceutical composition comprising at least one said therapeutic agent for use according to any one of claims 1 to 6, or at least one said expression vector according to claim 7.

**10.** The pharmaceutical composition according to claim 9, further comprising at least one additional anticancer agent in combination, either sequentially or simultaneously.

**11.** The pharmaceutical composition according to any one of claims 9 to 10, further comprising at least one pharmaceutically acceptable carrier.

**12.** A kit comprising :

a) At least one therapeutic agent for use according to any one of claims 1-6; and/or
b) At least one expression vector according to claim 7; and/or
c) At least one host cell according to claim 8; and/or
d) A pharmaceutical composition according to any one of claims 9 to 11.

**13.** A screening method for a therapeutic agent for use in the treatment of disease associated with cellular metabolism reprogramming comprising the steps of:

a) Providing SNAT7 protein;
b) Contacting said SNAT7 protein with a therapeutic agent candidate;
c) Determining the effect of said therapeutic agent candidate on the SNAT7 protein glutamine transport activity;
d) Selecting said therapeutic agent candidate if it significantly reduces or inhibits SNAT7 protein glutamine transport activity as determined in step c).

**14.** A screening method for a therapeutic agent according to claim 13, wherein said disease associated with cellular metabolism reprogramming is a cancer-associated with cellular metabolism reprogramming, preferentially is a cancer depending on glutamine.

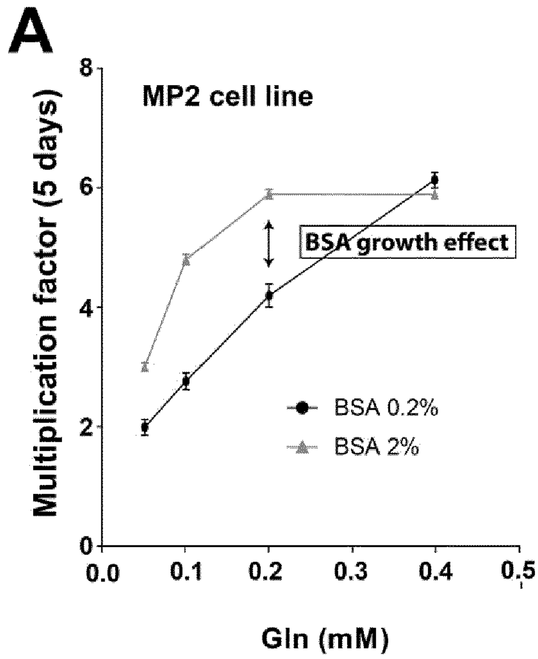
A
MP2 cell line
Multiplication factor (5 days)
BSA growth effect
BSA 0.2%
BSA 2%
Gln (mM)
0.0
0.1
0.2
0.3
0.4
0.5
0
2
4
6
8

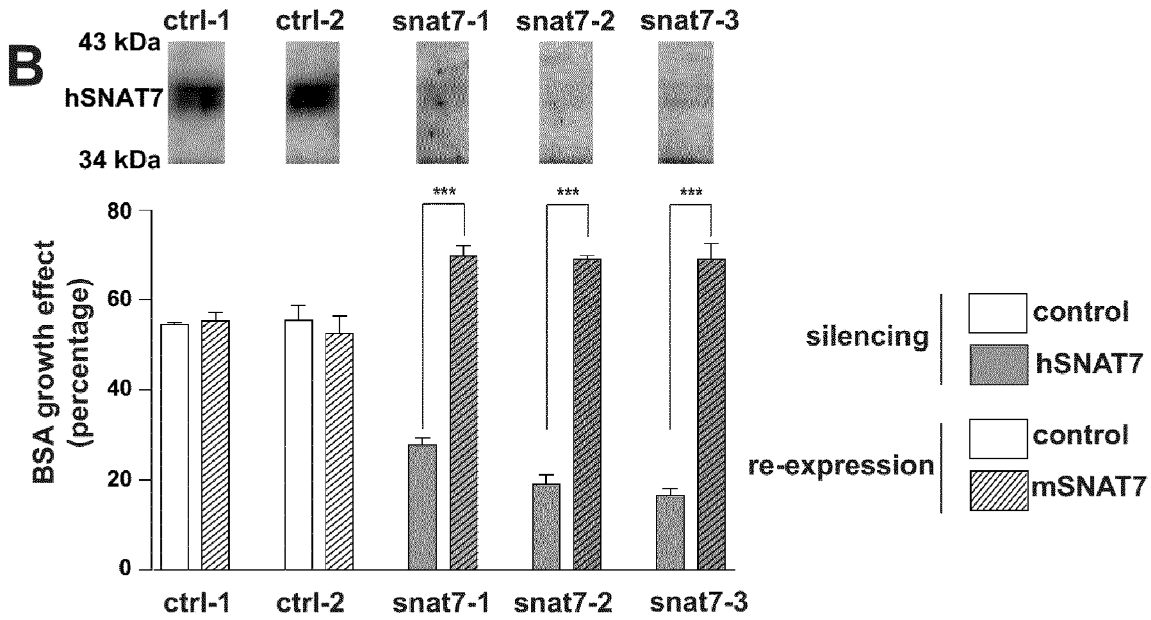
B
ctrl-1
ctrl-2
snat7-1
snat7-2
snat7-3
43 kDa
hSNAT7
34 kDa
BSA growth effect (percentage)
0
20
40
60
80
***
silencing
control
hSNAT7
re-expression
control
mSNAT7

Fig. 1

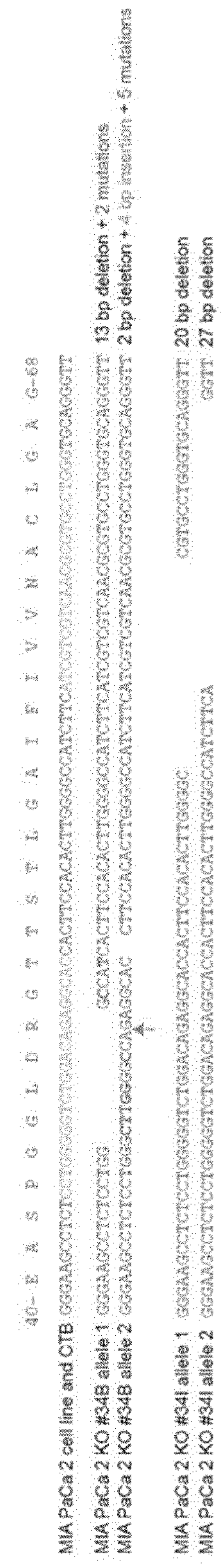


C

Fig. 1 (continued)

D

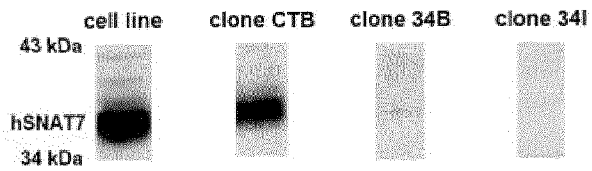


E

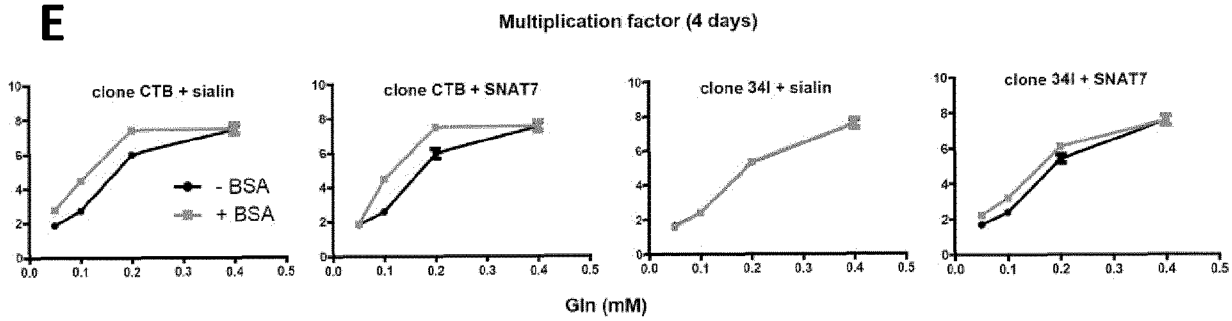


F

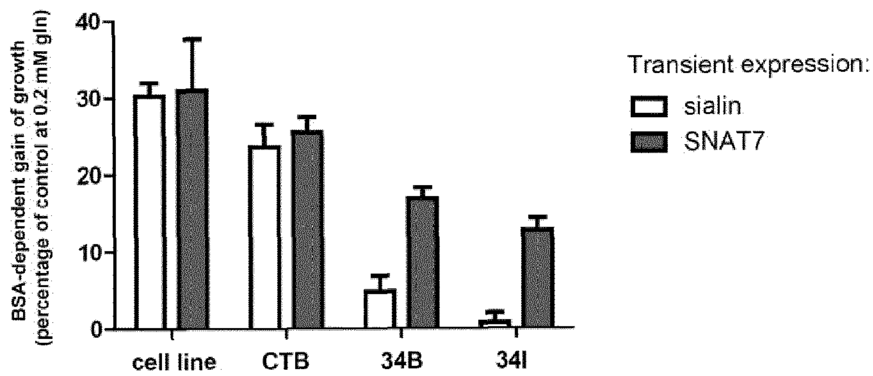


Fig. 1 (continued)

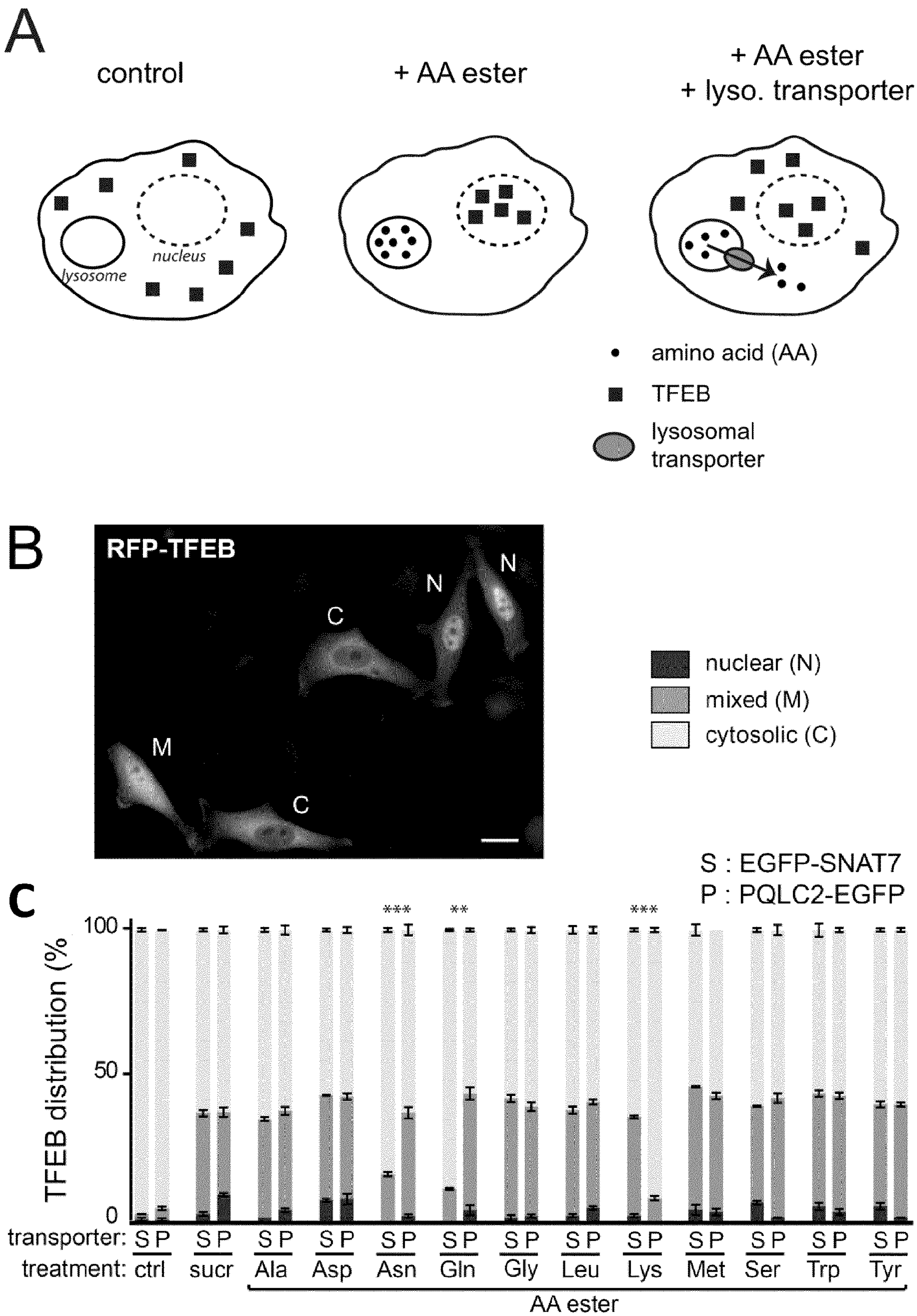
A
control
+ AA ester
+ AA ester
+ lyso. transporter
nucleus
lysosome
amino acid (AA)
TFEB
lysosomal transporter
B
RFP-TFEB
N
N
C
M
C
nuclear (N)
mixed (M)
cytosolic (C)
C
S : EGFP-SNAT7
P : PQLC2-EGFP
TFEB distribution (%)
100
50
0
***
**
***
transporter: S P S P S P S P S P S P S P S P S P S P S P S P S P
treatment: ctrl sucr Ala Asp Asn Gln Gly Leu Lys Met Ser Trp Tyr
AA ester

Fig. 2

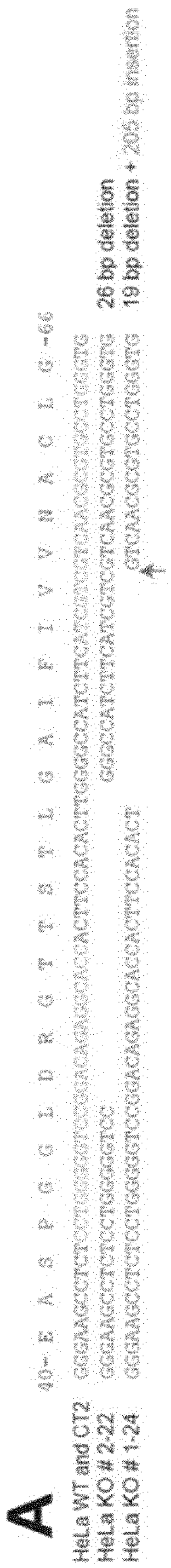
A
HeLa WT and CT2
HeLa KO # 2-22
HeLa KO # 1-24
26 bp deletion
19 bp deletion + 205 bp insertion

Fig. 3

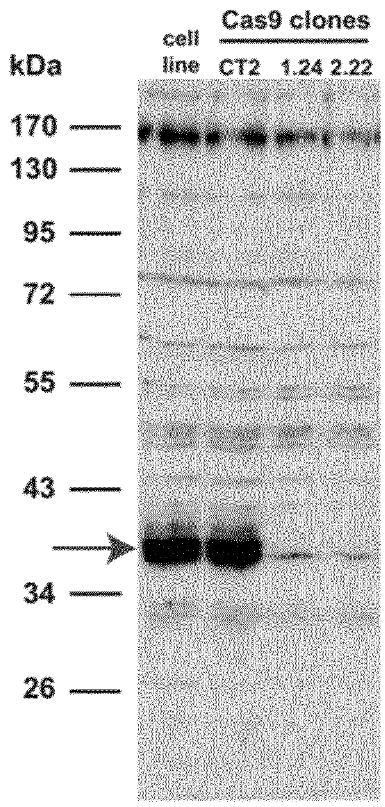


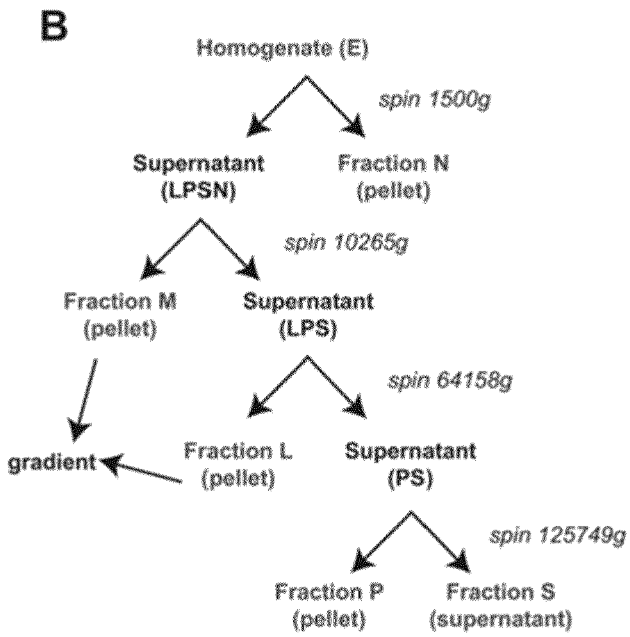


Fig. 3 (continued)

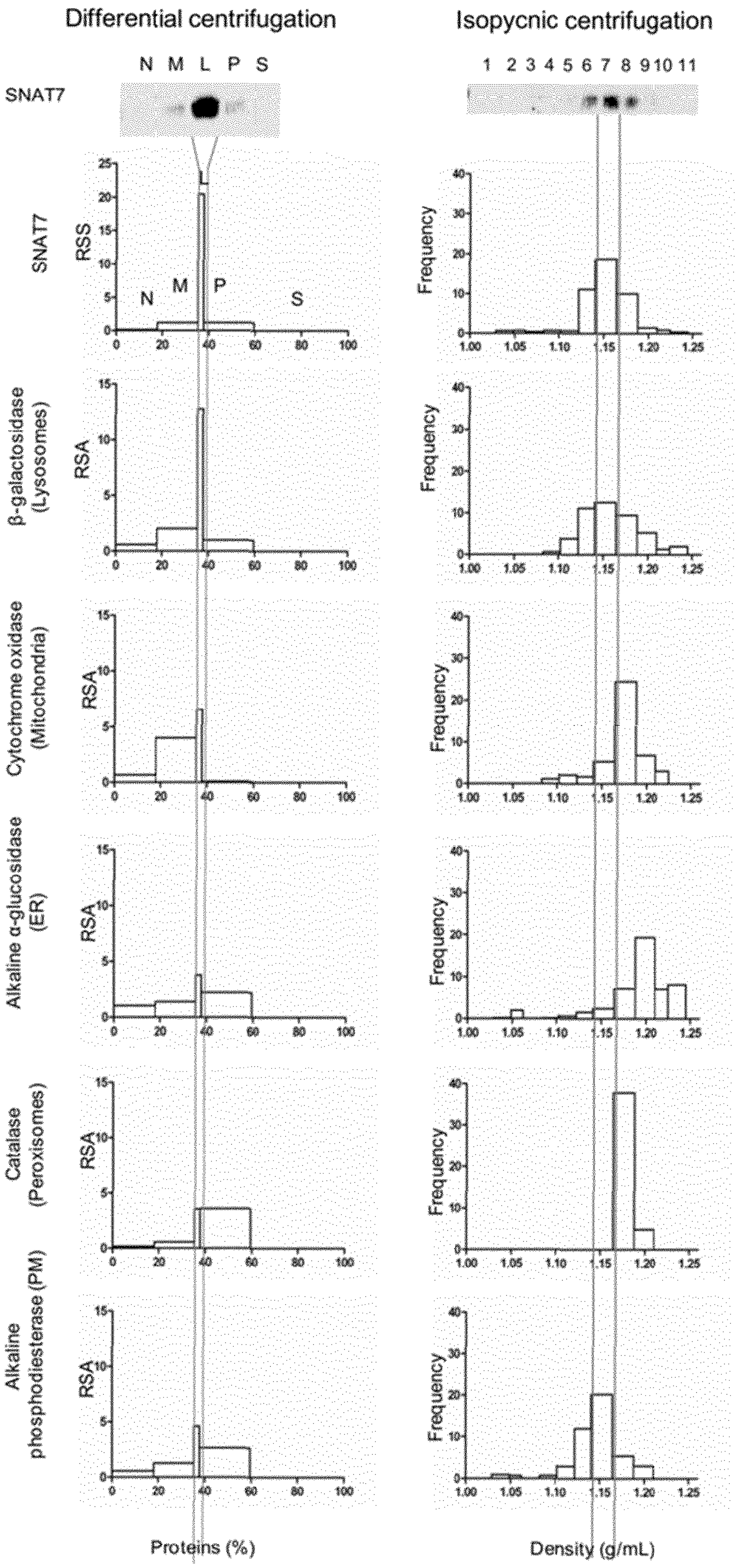


Fig. 3 (continued)

C

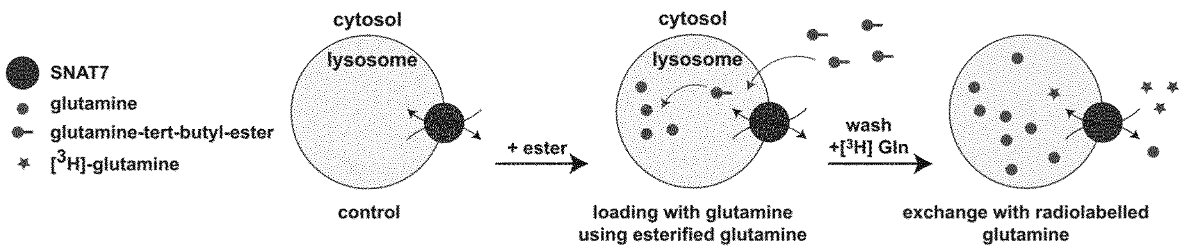


D

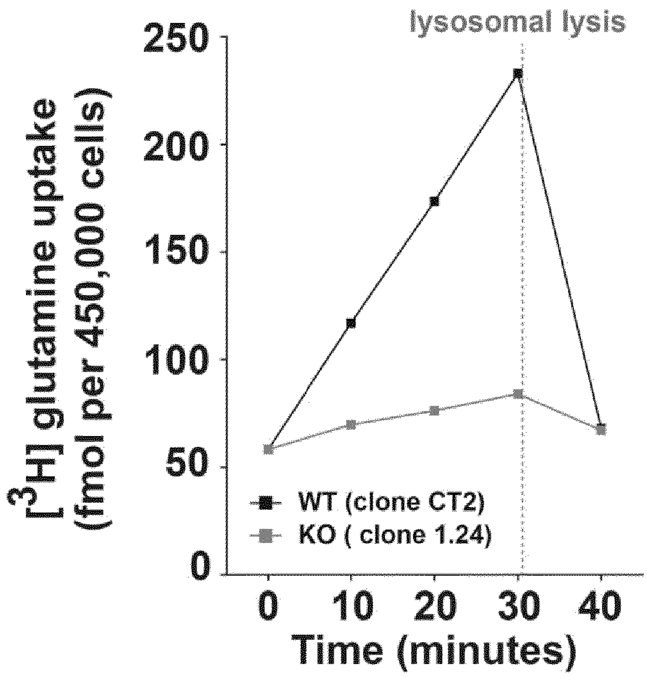


Fig. 3 (continued)

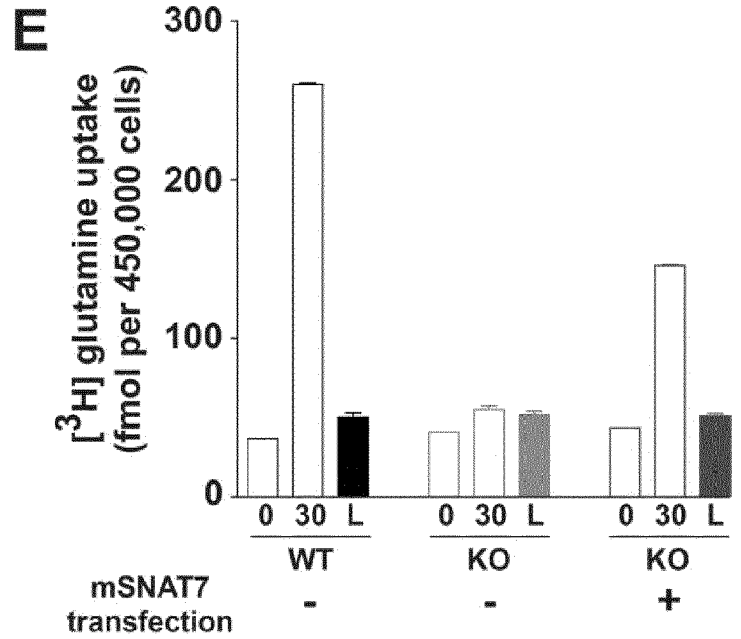


Fig. 3 (continued)

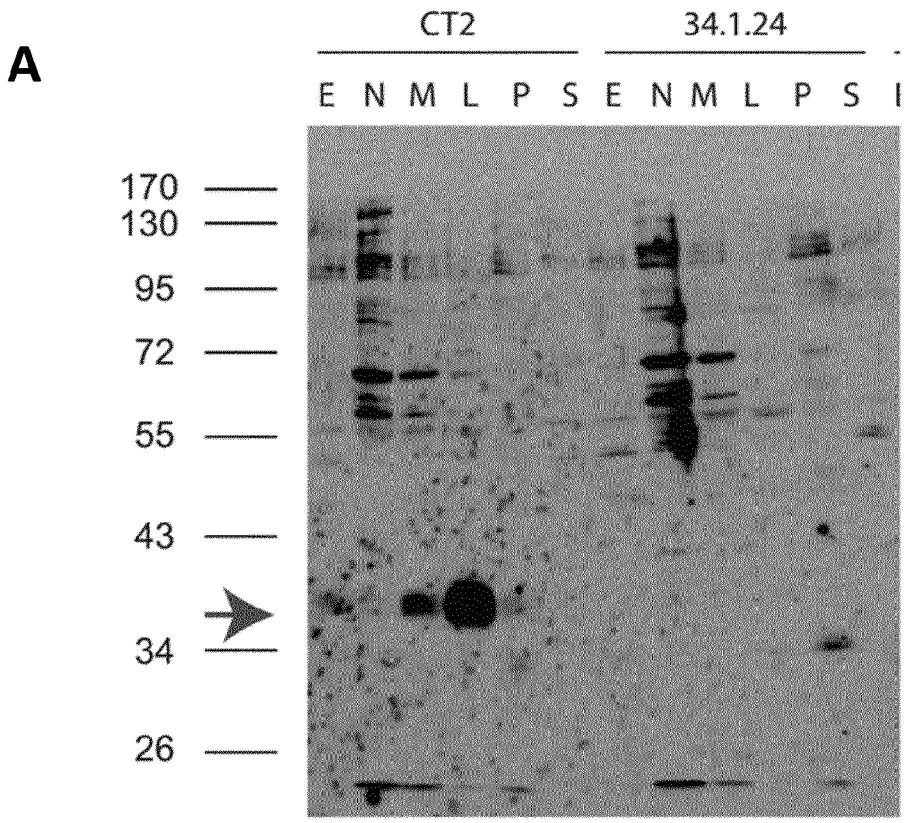


Fig. 4

B

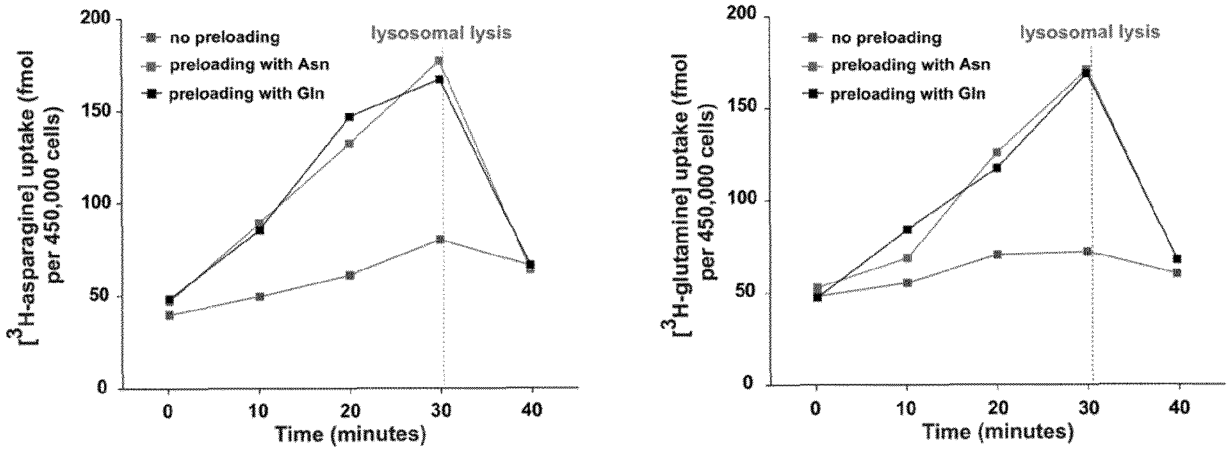


C

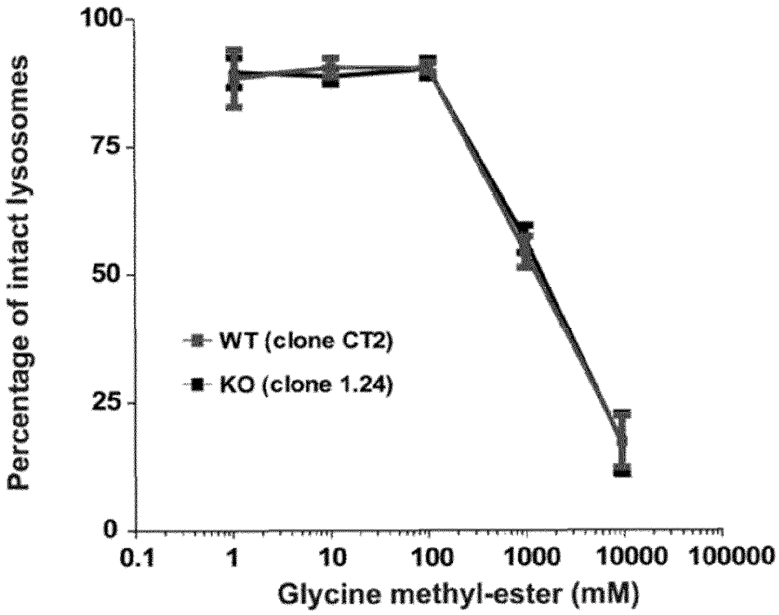


Fig. 4 (continued)

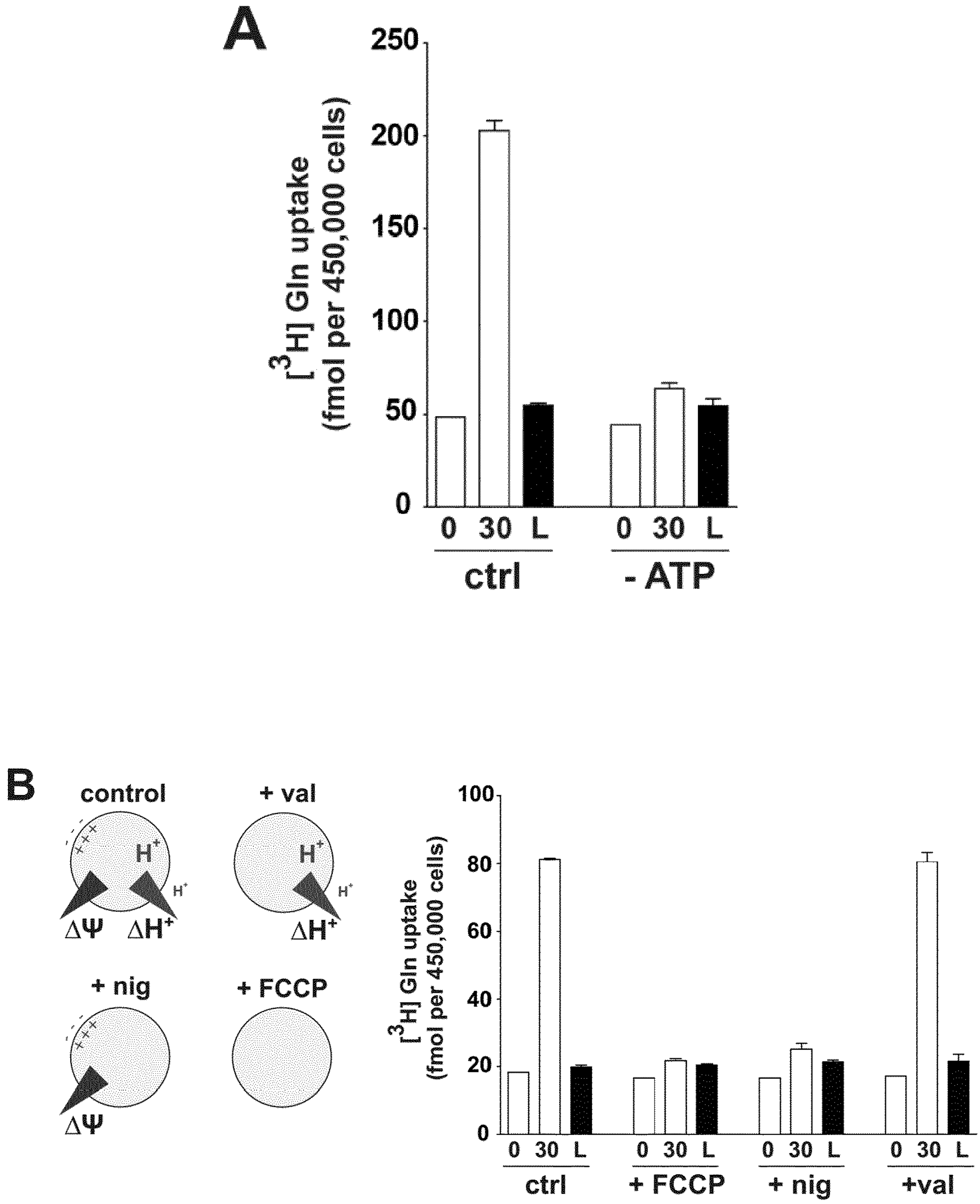
A
250
200
150
100
50
0
[3H] Gln uptake
(fmol per 450,000 cells)
0 30 L
0 30 L
ctrl
- ATP
B
control
+ val
H+
ΔΨ
ΔH+
+ nig
+ FCCP
100
80
60
40
20
0
ctrl
+ FCCP
+ nig
+val

Fig. 5

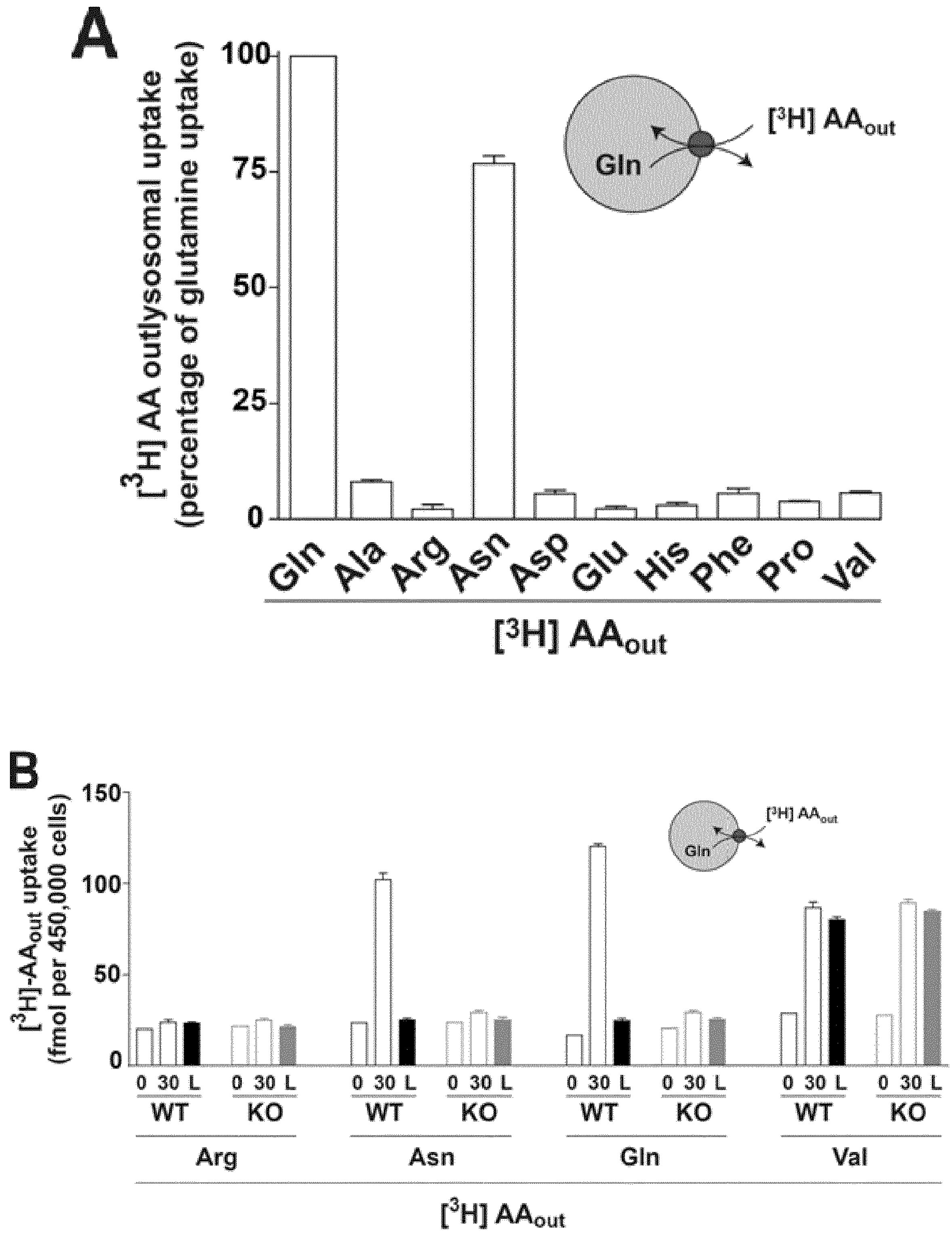
A
[3H] AA outlysosomal uptake (percentage of glutamine uptake)
100
75
50
25
0
Gln
Ala
Arg
Asn
Asp
Glu
His
Phe
Pro
Val
[3H] AAout
Gln
[3H] AAout
B
[3H]-AAout uptake (fmol per 450,000 cells)
150
100
50
0
0 30 L
WT
KO
Arg
Asn
Gln
Val
[3H] AAout

Fig. 6

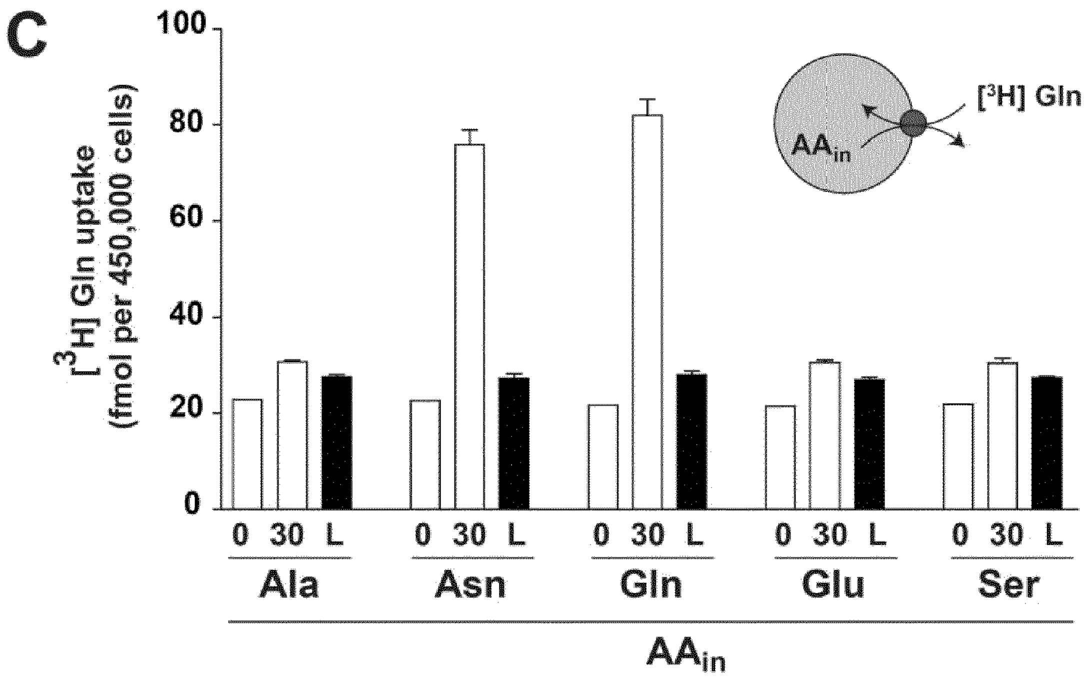


Fig. 6 (continued)

Europäisches Patentamt
European Patent Office
Office européen des brevets

# EUROPEAN SEARCH REPORT

Application Number
EP 16 30 5785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2016/007647 A1 (THE JOHNS HOPKINS UNIVERSITY [US]) 14 January 2016 (2016-01-14) * the whole document * ----- | 1-14 | INV. C12N15/113 C07K16/28 |
| A | M. BOLZONI ET AL.: "Dependence on glutamine uptake and glutamine addiction characterize myeloma cells: a new attractive target", BLOOD, vol. 128, no. 5, 6 June 2016 (2016-06-06), pages 667-679, XP055324555, US ISSN: 0006-4971, DOI: 10.1182/blood-2016-01-690743 * the whole document * ----- | 1-14 | |
| A | M. HASSANEIN ET AL.: "SLC1A5 mediates Glutamine transport required for lung cancer cell growth and survival", CLINICAL CANCER RESEARCH, vol. 19, no. 3, 1 February 2013 (2013-02-01), pages 560-570, XP055324559, US ISSN: 1078-0432, DOI: 10.1158/1078-0432.CCR-12-2334 * the whole document * ----- -/-- | 1-14 | TECHNICAL FIELDS SEARCHED (IPC): C12N C07K |

The present search report has been drawn up for all claims

| Place of search | Date of completion of the search | Examiner |
|---|---|---|
| The Hague | 30 November 2016 | Macchia, Giovanni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C01)

page 1 of 3

Europäisches Patentamt
European Patent Office
Office européen des brevets

# EUROPEAN SEARCH REPORT

Application Number
EP 16 30 5785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | M. CHIU ET AL.: "Glutamine depletion by crisantaspase hinders the growth of human hepatocellular carcinoma xenografts", BRITISH JOURNAL OF CANCER, vol. 111, no. 6, 9 September 2014 (2014-09-09), pages 1159-1167, XP055324566, GB ISSN: 0007-0920, DOI: 10.1038/bjc.2014.425 * the whole document * ----- | 1-14 | |
| A | WILLEMS LISE ET AL: "Inhibiting glutamine uptake represents an attractive new strategy for treating acute myeloid leukemia.", BLOOD, vol. 122, no. 20, 14 November 2013 (2013-11-14), pages 3521-3532, XP55324567, ISSN: 1528-0020 * the whole document * ----- | 1-14 | TECHNICAL FIELDS SEARCHED (IPC) |
| A | KOICHI SUGIMOTO ET AL.: "A clinically attainable dose of L-asparaginase targets glutamine addiction in lymphoid cell lines", CANCER SCIENCE, vol. 106, no. 11, 16 October 2015 (2015-10-16), pages 1534-1543, XP055324570, JP ISSN: 1347-9032, DOI: 10.1111/cas.12807 * the whole document * ----- | 1-14 | |
| | -/-- | | |

The present search report has been drawn up for all claims

1

| Place of search | Date of completion of the search | Examiner |
|---|---|---|
| The Hague | 30 November 2016 | Macchia, Giovanni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 30 5785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | M. I. GROSS ET AL.: "Antitumor activity of the Glutaminase inhibitor CB-839 in triple-negative breast cancer", MOLECULAR CANCER THERAPEUTICS, vol. 13, no. 4, 12 February 2014 (2014-02-12), pages 890-901, XP55243589, US ISSN: 1535-7163, DOI: 10.1158/1535-7163.MCT-13-0870 * the whole document * | 1-14 | |
| A | N. JACQUE ET AL.: "Targeting glutaminolysis has antileukemic activity in acute myeloid leukemia and synergizes with BCL-2 inhibition", BLOOD, vol. 126, no. 11, 17 July 2015 (2015-07-17), pages 1346-1356, XP55318334, US ISSN: 0006-4971, DOI: 10.1182/blood-2015-01-621870 * the whole document * | 1-14 | |

TECHNICAL FIELDS SEARCHED (IPC)

The present search report has been drawn up for all claims

| Place of search | Date of completion of the search | Examiner |
|---|---|---|
| The Hague | 30 November 2016 | Macchia, Giovanni |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 16 30 5785

This annex lists the patent family members relating to the patent documents cited in the above-mentioned European search report. The members are as contained in the European Patent Office EDP file on The European Patent Office is in no way liable for these particulars which are merely given for the purpose of information.

30-11-2016

| Patent document cited in search report | Publication date | Patent family member(s) | Publication date |
|---|---|---|---|
| WO 2016007647 A1 | 14-01-2016 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9119813 A
- US 5641870 A


### Non-patent literature cited in the description


- **JINEK, M. et al.** *Science,* 2012, vol. 337 (6096), 816-21
- **MALI, P. et al.** *Science,* 2013, vol. 339 (6121), 823-6
- **COMMISSO et al.** *Nature,* 2013
- **DUVE et al.** *Biochem. J,* 1955
- **WATTIAUX.** *Biochem J,* 2010
- **GASINGIRWA.** *Biochem J,* 2010
- **PUISSANT.** *Traffic,* 2014
- **BEAUFAY.** *J. Cell Biol.,* 1974
- *Delta Valle, Mol Cell Proteome,* 2011
- **DANG CV ; LE A ; GAO P.** MYC-induced cancer cell energy metabolism and therapeutic opportunities. *Clin Cancer Res.,* 2009, vol. 15 (21), 6479-83
- **BOROUGHS, L. K.,DEBERARDINIS, R. J.** Metabolic pathways promoting cancer cell survival and growth. *Nature Cell Biology,* 2015, vol. 17 (4), 351-359
- **BHUTIA YD ; BABU E ; RAMACHANDRAN S ; GANAPATHY V.** Amino Acid transporters in cancer and their relevance to "glutamine addiction": novel targets for the design of a new class of anticancer drugs. *Cancer Res.,* 2015, vol. 75 (9), 1782-8
- **BRÖER A ; RAHIMI F ; BRÖER S.** Deletion of Amino Acid Transporter ASCT2 (SLC1A5) Reveals an Essential Role for Transporters SNAT1 (SLC38A1) and SNAT2 (SLC38A2) to Sustain Glutaminolysis in Cancer Cells. *J Biol Chem.,* 2016, vol. 291 (25), 13194-205
- **CADORET A ; OVEJERO C ; TERRIS B ; SOUIL E ; LEVY L ; LAMERS WH ; KITAJEWSKI J ; KAHN A ; PERRET C.** New targets of beta-catenin signaling in the liver are involved in the glutamine metabolism. *Oncogene,* 28 November 2002, vol. 21 (54), 8293-301
- **COMMISSO C ; DAVIDSON SM ; SOYDANER-AZELOGLU RG ; PARKER SJ ; KAMPHORST JJ ; HACKETT S ; GRABOCKA E ; NOFAL M ; DREBIN JA ; THOMPSON CB.** Macropinocytosis of protein is an amino acid supply route in Rastransformed cells. *Nature,* 2013, vol. 497 (7451), 633-7
- **FOLKMAN J ; SHING Y.** *Angiogenesis J Biol Chem.,* 05 June 1992, vol. 267 (16), 10931-4
- **HAGGLUND MG ; SREEDHARAN S ; NILSSON VC ; SHAIK JH ; ALMKVIST IM ; BÄCKLIN S ; WRANGE 0 ; FREDRIKSSON R.** Identification of SLC38A7 (SNAT7) protein as a glutamine transporter expressed in neurons. *J Biol Chem.,* 2011, vol. 286 (23), 20500-11
- **HAGGLUND MG ; HELLSTEN SV ; BAGCHI S ; PHILIPPOT G ; LÖFQVIST E ; NILSSON VC ; ALMKVIST I ; KARLSSON E ; SREEDHARAN S ; TAFRESHIHA A.** Transport of L-glutamine, L-alanine, L-arginine and L-histidine by the neuron-specific Slc38a8 (SNAT8) in CNS. *J Mol Biol.,* 2015, vol. 427 (6), 1495-512
- **PAVLOVA NN.** ThompsonCB. The Emerging Hallmarks of Cancer Metabolism. *Cell Metab.,* 12 January 2016, vol. 23 (1), 27-47
- **STROHECKER, A. M. ; GUO, J. Y. ; KARSLI-UZUNBAS, G. ; PRICE, S. M. ; CHEN, G. J. ; MATHEW, R. ; WHITE, E.** Autophagy sustains mitochondrial glutamine metabolism and growth of BrafV600E-driven lung tumors. *Cancer Discovery,* 2013, vol. 3 (11), 1272-85, http://doi.org/10.1158/2159-8290.CD-13-0397
- **TIMMERMAN LA ; HOLTON T ; YUNEVA M ; LOUIE RJ ; PADRÓ M ; DAEMEN A ; HU M ; CHAN DA ; ETHIER SP ; VAN 'T VEER LJ.** Glutamine sensitivity analysis identifies the xCT antiporter as a common triple-negative breast tumor therapeutic target. *CANCER CELL.,* 14 October 2013, vol. 24 (4), 450-65
- **WEINSTEIN IB ; JOE A.** *Oncogene addiction Cancer Res.,* 01 May 2008, vol. 68 (9), 3077-80
- **BRÖER, S.** The SLC38 family of sodium-amino acid co-transporters. *Pflugers Archiv European Journal of Physiology,* 2014, vol. 466, 155-172, http://doi.org/10.1007/s00424-013-1393-y
- **WANG, S., TSUN, Z., WOLFSON, R., SHEN, K., WYANT, G., PLOVANICH, M., ... SABATINI, D.** *Lysosomal amino acid transporter SLC38A9 signals arginine sufficiency to mTORC1,* 1995, vol. 347 (6218), 188-194

- **REBSAMEN, M. ; POCHINI, L. ; STASYK, T. ; DE ARAÚJO, M. E. G. ; GALLUCCIO, M. ; KANDASAMY, R. K. ; SUPERTI-FURGA, G.** SLC38A9 is a component of the lysosomal amino acid sensing machinery that controls mTORC1. *Nature,* 2015, vol. 519 (7544), 477-81, http://doi.org/10.1038/nature14107
- **CHRISTENSEN HN.** Role of amino acid transport and countertransport in nutrition and metabolism. *Physiol Rev.,* January 1990, vol. 70 (1), 43-77
- **JUNG, J. ; GENAU, H. M. ; BEHRENDS, C.** Amino Acid-Dependent mTORC1 Regulation by the Lysosomal Membrane Protein SLC38A9. *Molecular and Cellular Biology,* 2015, vol. 35 (14), 2479-2494
- **RAN, F. A., HSU, P. D., WRIGHT, J., AGARWALA, V., SCOTT, D. A, ZHANG, F.** Genome engineering using the CRISPR-Cas9 system. *Nature Protocols,* 2013, vol. 8 (11), 2281-308, http: //doi.org/10.1038/nprot.2013.143
- **JHA, A. K. ; HUANG, S. C. C. ; SERGUSHICHEV, A. ; LAMPROPOULOU, V. ; IVANOVA, Y. ; LOGINICHEVA, E. ; ARTYOMOV, M. N.** Network integration of parallel metabolic and transcriptional data reveals metabolic modules that regulate macrophage polarization. *Immunity,* 2015, vol. 42 (3), 419-430, http://doi.org/10.1016/j.immuni.2015.02.005